# EUROPÄISCHE PATENTSCHRIFT

(11) **EP 0 765 871 B1**
(45) Veröffentlichungstag und Bekanntmachung des Hinweises auf die Patenterteilung: **31.03.2004**
(21) Anmeldenummer: 96115110.7
(22) Anmeldetag: 20.09.1996
(51) Int. Cl.: C07D 215/60, C07D 215/16, C07D 215/48, A61K 31/47

(54) **Substituierte Chinolin-2-Carbonsäureamide, ihre Herstellung und ihre Verwendung als Prolyl-4-hydroylase Inhibitoren**
Substituted Quinoline-2-carboxamides, their preparation and their use as prolyle-4-hydroxylase Inhibitors
Dérivés de quinoline-2-carboxamide, leur préparation et leur utilisation comme inhibiteur de la prolyl-4-hydroxylase

(30) Priorität: 28.09.1995 DE 19536263; 13.02.1996 DE 19605170
(43) Veröffentlichungstag der Anmeldung: 02.04.1997
(73) Patentinhaber: FibroGen, Inc., South San Francisco, CA 94080 (US)
(72) Erfinder: Weidmann, Klaus, Dr., 61476 Kronberg (DE); Baringhaus, Karl-Heinz, Dr., 61200 Wölfersheim (DE); Tschank, Georg, Dr., 55270 Klein-Winterheim (DE); Bickel, Martin, Dr., 61348 Bad Homburg (DE)
(74) Vertreter: Janssen, Bernd Christian

(56) Entgegenhaltungen:
- EP-A- 0 562 512
- EP-A- 0 590 520
- EP-A- 0 650 960
- EP-A- 0 650 961
- EP-A- 0 661 269
- BIOCHEM.SOC.TRANS., Bd. 19, 1991, Seiten 812-815, XP002022664 FRANKLIN,J.F. ET AL.: "Approaches to the design of anti-fibrotic drugs"
- J.AM.CHEM.SOC., Bd. 68, 1946, WASHINGTON, Seiten 1232-1238, XP002022665 BRESLOW,D.S. ET AL.: "Synthesis of Antimalerials. V. The Syntheis of Certain 4-Aminoquinoline Derivatives"
- CHEM.PHARM.BULL., Bd. 15, Nr. 5, 1967, TOKYO, Seiten 663-669, XP002022666 KANEKO,C. ET AL.: "The Isomerisation of 1aH-Oxazirino[2,3-a]quinoline 1a-carbonitrile ...."
- GAZZ.CHIM.ITAL., Bd. 101, 1971, ROM, Seiten 104-105, XP002022667 FATTORUSSO,E. ET AL.: "Isolation of 3,4-dihydroxyquinoline2-carboxylic acid from the sponge Aplysina aerophoba"
- J.CHEM.SOC. C, Nr. 4, 1967, Seiten 256-261, XP002022668 HANNAH,E.D. ET AL.: "Removal of tolyl-sulphonyl groups from sulfonamides. III. some N-(tolyl-p-sulphonyl) glycine esters"
- J.AM.CHEM.SOC., Bd. 115, 1993, WASHINGTON, Seiten 11624-11625, XP002022669 BOGER,D.L. ET AL.: "(-)Sandramycin: Totoal Syntesei and Preliminary DNA Binding Properties"
- J.AM.CHEM.SOC., Bd. 103, Nr. 5, 1981, WASHINGTON, Seiten 1241-1243, XP002022670 KONISHI,M. ET AL.: "Structures of BBM-928 A,B and C...."
- J.CHEM.SOC.,CHEM.COMMUM., Nr. 19, 1975, LONDON, Seiten 782-783, XP002022671 BAYNE,D.W.ET AL.: "Synthesis of 2-Acyl-3-hydroxyquinolines Embodying a Novel Variant of the Smiles Rearrangement "

## Beschreibung

Die Erfindung betrifft substituierte Chinolin-2-carbonsäureamide, ihre Herstellung und ihre Verwendung als Inhibitoren der Prolyl-4-hydroxylase und ihre Verwendung als Arzneimittel zur Behandlung von fibrotischen Erkrankungen sowie Zwischenprodukte bei deren Herstellung.

Verbindungen, die die Enzyme Prolyl- und Lysylhydroxylase inhibieren, bewirken eine sehr selektive Hemmung der Kollagenbiosynthese durch Beeinflussung der kollagenspezifischen Hydroxylierungsreaktionen. In deren Verlauf wird proteingebundenes Prolin oder Lysin durch die Enzyme Prolyl- bzw. Lysylhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Inhibitoren der Prolylhydroxylase sind deshalb geeignete Substanzen in der Therapie von Erkrankungen, in denen die Ablagerung von Kollagenen maßgeblich zum Krankheitsbild beiträgt. Hierzu gehören u. a. Fibrosen der Lunge, Leber und Haut (Skleroderma und Vernarbungen nach Verbrennungen, Verletzungen und chirurgischen Eingriffen) und sowie die Atherosklerose.

Es ist bekannt, daß das Enzym Prolylhydroxylase durch Pyridin-2,4- und -2,5-dicarbonsäure effektiv gehemmt wird (K. Majamaa et al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 238 (1987) 625 bis 633).

Auch Prodrugs der Pyridin-2,4(5)-dicarboxylate sind bekannt. Diese sind in den EP-A-0 590 520 und EP-A-0 562 512 beschrieben.

N-Oxalylglycine als Inhibitoren der Prolyl-4-hydroxylase sind aus J. Med. Chem. 1992, 35, 2652 bis 2658 (Cunliffe et al.), und EP-A-0 457 163 (Baader et al.) bekannt.

3-Hydroxypyridin-2-carbonsäure-N-(carboxymethyl)amid ist aus G. Yolles et al. in: Bull. Soc. Chim. Fr. 1965, 8, 2252 bis 2259 bekannt.

Hydroxyisochinolin- und Hydroxycinnolincarbonsäureglycylamide sind aus Biochem. Soc. Trans. 1991, 19, 812 bis 815 (Franklin et al.) bekannt.

In der EP-A-0 661 269 werden substituierte heterocyclische Carbonsäureamide und ihre Verwendung als Inhibitoren der Prolyl-4-hydroxylase und als Hemmstoffe der Kollagenbiosynthese beschrieben.

Es bestand die Aufgabe, nach noch wirksameren Inhibitoren der Prolylhydroxylase und nach weiteren Hemmstoffen der Kollagenbiosynthese zu suchen.

Es wurde nun gefunden, daß eine Auswahl von den von der EP-A-0 661 269 umfaßten Verbindungen, nämlich die Chinolin-2-carbonsäureamide mit einer OH-Funktion in ortho-Position zur Amidfunktion eine überraschend hohe Hemmung der Prolyl-4-hydroxylase in Zellkulturen aufweisen.

Die erfindungsgemäßen Verbindungen entsprechen der allgemeinen Formel (I) 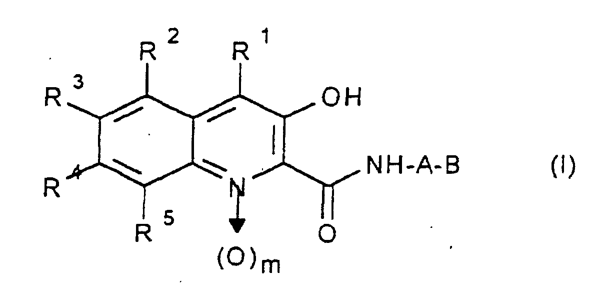 in welcher
- m: 0
- A: eine -CH₂-Gruppe, die mit einer Methylgruppe substituiert sein kann,
- B: -CO₂H,
- R¹: Wasserstoff, (C₁-C₄)-Alkyl, (C₁-C₄)-Aloxy, Chlor, Brom,
- R⁵: Wasserstoff, Fluor, Chlor, Methyl bedeutet,
- R²: und R³ gleich oder verschieden sind und Wasserstoff, (C₁-C₁₈)-Alkyl, (C₂-C₁₈)-Alkenyl, (C₂-C₁₈)-Alkinyl, Phenyl, Chlor, Brom, Hydroxy, Triflormethyl, (C₁-C₁₈)-Alkylsulfinyl, (C₁-C₁₈)-Alkylsulfonyl, Phenylsulfinyl, Phenylsulfonyl, Naphtylsulfinyl, Naphtylsulfonyl, (C₁-C₁₈)-Alkoxy, (C₃-C₈)-Cycloalkxy, (C₁-C₈)-Alkoxy-(C₁-C₈)-alkoxy, -O-[CH₂]ₓ-C_{f}H_{(2f+1-g)}F_{g}, Phenyl- (C₁-C₄)-alkoxy, Phenoxy, (C₁-C₁₂)-Alkanoyl, Phenyl-(C₁-C₄)-alkanoyl oder. Benzoyl bedeuten, wobei in Substituenten mit einem Phenyl- oder Naphtyl- Ring dieser gegebenenfalls bis zu 5 gleiche oder verschiedene Substituenten aus der Reihe Fluor, Chlor, Brom, Nitril, Trifluormetyl, (C₁-C₆)-Alkyl, (C₁-C₆)-Alkoxy, -O-[CH₂]ₓ-C_{f}H_{(2f+1-g)}F_{g} oder (C₁-C₆)-Alkylsulfonyl trägt,
- R⁴: Flour, Chlor, Brom bedeutet
- f: 1 - 8
- g: 0, 1 bis (2f+1)
- x: 0 bis 3 bedeuten, einschließlich der physiologisch wirksamen Salze.

Die Erfindung umfaßt weiterhin Salze der Verbindungen der allgemeinen Formel I.

Die Salzbildung mit basischen Reagenzien kann ein- oder zweifach an den aciden Gruppen der Verbindungen der Formel I, d.h. an den Resten B, R¹, R², R³, R⁴ und R⁵ und/oder an der aciden phenolischen OH-Gruppe, insbesondere an den Resten B und der phenolischen OH-Gruppe erfolgen.

Zur Anwendung kommende Reagenzien sind beispielsweise Alkoholate, Hydroxide, Carbonate, Hydrogencarbonate, Hydrogenphosphate und/oder Metallorganyle der Alkali- und Erdalkalielemente, der Elemente der 3. und 4. Hauptgruppe des Periodensystems und der Elemente der Übergangsmetalle,

Amine, gegebenenfalls 1- bis 3-fach substituiert mit (C₁-C₈)-Hydroxyalkyl, (C₁-C₄)-Alkoxy-(C₁-C₈)-alkyl, Phenyl, Benzyl oder (C₁-C₈)-Alkyl, welches 1- bis 3-fach substituiert sein kann mit Hydroxy oder (C₁-C₄)-Alkoxy, beispielsweise Tromethan (Tris-Puffer), 2-Aminoethanol, 3-Aminopropanol, Hydroxylamin, Dimethylhydroxylamin, 2-Methoxy-ethylamin, 3-Ethoxypropylamin, und
basische Aminosäuren und -derivate, wie Aminosäureester, Histidin, Arginin und Lysin und deren Derivate, sowie

Arzneimittel, die eine basische Gruppe enthalten, wie beispielsweise Amilorid, Verapamil und Betablocker.

Die Erfindung betrifft weiterhin die Verbindungen gemäß Formel I, zur Anwendung als Arzneimittel.

Ganz besonders bevorzugt sind Verbindungen der Formel I, in der
- m: 0,
- A: eine -CH₂-Gruppe,
- B: -CO₂H,
- R¹ und R⁵: Wasserstoff,
- R² und R³: gleich oder verschieden sind und Wasserstoff, (C₁-C₁₈)-Alkyl, (C₁-C₁₈)-Alkenyl, Phenyl, Chlor, Fluor, Brom, Trifluormethyl, (C₁-C₁₈)-Alkylsulfinyl, (C₁-C₁₈)-Alkylsulfonyl, Phenylsulfinyl, Phenylsulfonyl, Naphthylsulfinyl, Naphthylsulfonyl, (C₁-C₁₈)-Alkoxy, (C₃-C₈)-Cycloalkoxy, (C₁-C₈)-Alkoxy-(C₁-C₈)-alkoxy, -O-[CH₂]ₓ-C_{f}H_{(2f+1-g)} F_{g}, Phenyl-(C₁-C₄)-alkoxy, Phenoxy, (C₁-C₁₂)-Alkanoyl, Phenyl-(C₁-C₄)-alkanoyl oder Benzoyl bedeuten, wobei in Substituenten mit einem Phenyl- oder Naphthyl-Ring dieser gegebenenfalls bis zu 5 gleiche oder verschiedene Substituenten aus der Reihe Fluor, Chlor, Brom, Nitril, Trifluormethyl, (C₁-C₆)-Alkyl, (C₁-C₆)-Alkoxy, -O-[CH₂]ₓ-C_{f}H_{(2f+1-g)} F_{g} oder (C₁-C₆)-Alkylsulfonyl trägt,
- R⁴: Fluor, Chlor, Brom bedeutet einschließlich der physiologisch wirksamen Salze.

Im Besonderen bevorzugt sind Verbindungen der Formel I, in der
- m: O,
- A: eine -CH₂-Gruppe,
- R¹ und R⁵: Wasserstoff,
einer der Substituenten R² oder R³ Wasserstoff bedeutet und der andere Wasserstoff, (C₁-C₁₆)-Alkyl, Fluor, Chlor, Brom, Trifluormethyl, (C₁-C₁₆)-Alkylsulfonyl, Phenylsulfonyl, (C₁-C₁₆)-Alkoxy, (C₁-C₈)-Alkoxy-(C₁-C₈)-alkoxy,-O-[CH₂]ₓ-C_{f}H_{(2f+1-g)}F_{g}, Benzyloxy oder Phenoxy bedeutet, wobei in Substituenten, die einen Phenylring enthalten, dieser gegebenenfalls bis zu 3 Substituenten aus der Reihe Fluor, Chlor, Brom, Trifluormethyl, (C₁-C₆)-Alkyl, (C₁-C ₆)-Alkoxy, -O-[CH₂]ₓ-C_{f}H_{(2f+1-g)}F_{g} oder (C₁-C₄)-Alkylsulfonyl, trägt,
R⁴ Fluor, Chlor, Brom, bedeutet, einschließlich der physiologisch wirksamen Salze.

Im Besonderen bevorzugt sind Verbindungen der Formel I, in der
- m: 0,
- A: eine -CH₂-Gruppe,
- R¹, R² und R⁵: Wasserstoff,
- R³: Wasserstoff, (C₁-C₁₆)-Alkyl, Fluor, Chlor, Trifluormethyl, (C₁-C₁₆)-Alkylsulfonyl, Phenylsulfonyl, Naphthylsulfonyl, (C₁-C₁₆)-Alkoxy, (C₁-C₈)-Alkoxy-(C₁-C₈)-alkoxy, -O-[CH₂]ₓ-C_{f}H_{(2f+1-g)}F_{g}, Benzyloxy oder Phenoxy bedeuten, wobei in Substituenten, die einen Phenylring enthalten, dieser gegebenenfalls bis zu 3 Substituenten aus der Reihe Fluor, Chlor, Trifluormethyl, (C₁-C₆)-Alkyl, (C₁-C₆)-Alkoxy, -O-[CH₂]ₓ-C_{f}H_{(2f+1-g)}F_{g} oder (C₁-C₄)-Alkylsulfonyl, trägt,
- R⁴: Fluor, Chlor, Brom bedeutet einschließlich der physiologisch wirksamen Salze.

Im Besonderen bevorzugt sind weiterhin Verbindungen der Formel I, in der
- m: 0,
- A: eine -CH₂-Gruppe,
- R¹, R², R³ und R⁵: Wasserstoff,
- R⁴: Fluor, Chlor, Brom, bedeutet, einschließlich der physiologisch wirksamen Salze.

Im Besonderen bevorzugt sind weiterhin Verbindungen der Formel I, in der
- m: 0,
- A: eine -CH₂-Gruppe,
- R¹, R² und R⁵: Wasserstoff,
- R⁴: Chlor,
- R³: Wasserstoff, Fluor, Chlor, (C₁-C₁₂)-Alkoxy, (C₁-C₄)-Alkoxy-(C₁-C₆)-alkoxy, - O-[CH₂]ₓ-C_{f}H_{(2f+1-g)}F_{g}, Phenylsulfonyl, Naphthylsulfonyl oder Phenoxy bedeutet, wobei in Substituenten, die einen Phenyl- oder Naphthyl-Ring enthalten, dieser gegebenenfalls bis zu 3 gleiche oder verschiedene, vorzugsweise einen Substituenten aus der Reihe Fluor, Chlor, Brom, Trifluormethyl, Trifluormethoxy, (C₁-C₆)-Alkyl und (C₁-C₆)-Alkoxy trägt, einschließlich der physiologisch wirksamen Salze.

Die Erfindung umfaßt weiterhin Prodrugs zu den Verbindungen der Formel (I), die eine Hemmung der Kollagenbiosynthese in vivo durch Freisetzung von Verbindungen der Formel I oder deren Salzen bewirken.

Schließlich umfaßt die Erfindung auch Prodrugs, die in vivo durch Freisetzung von Verbindungen der Formel I oder deren Salzen eine inhibitorische Wirkung auf die Prolyl-4-hydroxylase bewirken.

Prodrug-Gruppierungen sind chemische Gruppen, die in vivo
- zur Carboxylatgruppe der Verbindungen der Formel I umgewandelt werden und/oder
- vom Amid-N-Atom abgespalten werden können und/oder
- zu einem Chinolinring umgewandelt werden können.

Die in Betracht kommenden Prodrug-Gruppen sind dem Fachmann bekannt.

Insbesondere sind folgende Prodrug-Gruppierungen genannt:
für die Carboxylatgruppe Ester-, Amid-, Hydroxymethyl- und Aldehydgruppen und deren Abkömmlinge, für das Chinolin-N-Atom N-Oxide und N-Alkylderivate.

Die Erfindung betrifft die Verwendung von Verbindungen der allgemeinen Formel I sowie die physiologisch verträglichen Salze zur Herstellung eines Arzneimittels zur Inhibierung der Kollagenbiosynthese.

Die Erfindung betrifft die Verwendung von Verbindungen der allgemeinen Formel I sowie die physiologisch verträglichen Salze zur Herstellung eines Arzneimittels zur Hemmung der Prolyl-4-hydroxylase.

Weiterhin betrifft die Erfindung die Verwendung von Verbindungen der allgemeinen Formel I sowie die physiologisch verträglichen Salze zur Herstellung eines Arzneimittels gegen fibrotische Erkrankungen.

Weiterhin betrifft die Erfindung die Verwendung von Verbindungen der allgemeinen Formel I sowie die physiologisch verträglichen Salze zur Herstellung eines Arzneimittels gegen fibrotische Erkrankungen der Leber, der Lunge und der Haut.

Schließlich betrifft die Erfindung die Verbindungen der allgemeinen Formel I zur Verwendung als Arzneimittel.

Insbesondere betrifft die Erfindung die Verbindungen der Formel I zur Herstellung eines Arzneimittels zur Anwendung als Fibrosuppressiva.

Weiterhin betrifft die Erfindung ein Verfahren zur Herstellung von Verbindungen der allgemeinen Formel I.

Die Herstellung der Verbindungen der Formel I, in der
- A - B =: -(CH₂)₁₋₄-CO₂H und
- m =: 0 bedeuten, erfolgt, indem

1.i1.) Chinolin-2-carbonsäuren der Formel II (R¹¹ = H) mit den Aminoestern der Formel III zu den Amidestern der Formel IV umgesetzt werden, oder
1.i2.) Chinolin-2-carbonsäureester der Formel II (R¹¹ = niedrig Alkyl) unter den Bedingungen der Aminolyse zu den Verbindungen der Formel IV umgesetzt werden;
1.ii) die Verbindungen der Formeln I bzw. V aus ihren Estern der Formeln IV bzw. VI freigesetzt werden; und
1.iii) die Verbindungen der Formeln I bzw. VI aus den Verbindungen der Formeln V bzw. IV durch Abspaltung der Hydroxy-Schutzgruppe R¹⁰ erhalten werden und ggf.
1.iv) die Verbindungen der Formel I, IV, V oder VI zu Verbindungen der Formeln Ia, IVa, Va oder VIa oxidiert werden.

Geeignete Schutzgruppen, (PG = Protecting Groups) wie sie dem Fachmann geläufig sind, sind insbesondere Benzyl, p-Methoxybenzyl, 3,4-Dimethoxybenzyl, Alkyl, Methoxymethyl (MOM), Methylthio, Benzyloxymethyl (BOM), t-Butyloxymethyl, 2-Methoxyethoxymethyl (MEM) und Tetrahydropyranyl (THP).

Weitere Schutzgruppen und die Bedingungen ihrer Abspaltung (Überführung von Verbindungen der Formel V in Verbindungen der Formel I) sind von Theodoro W. Greene, Peter G.M. Wuts, in Protective Groups in Organic Synthesis, Second Edition 1991, John Wiley, Kapitel 2 und 3, Seiten 10 bis 174 beschrieben.

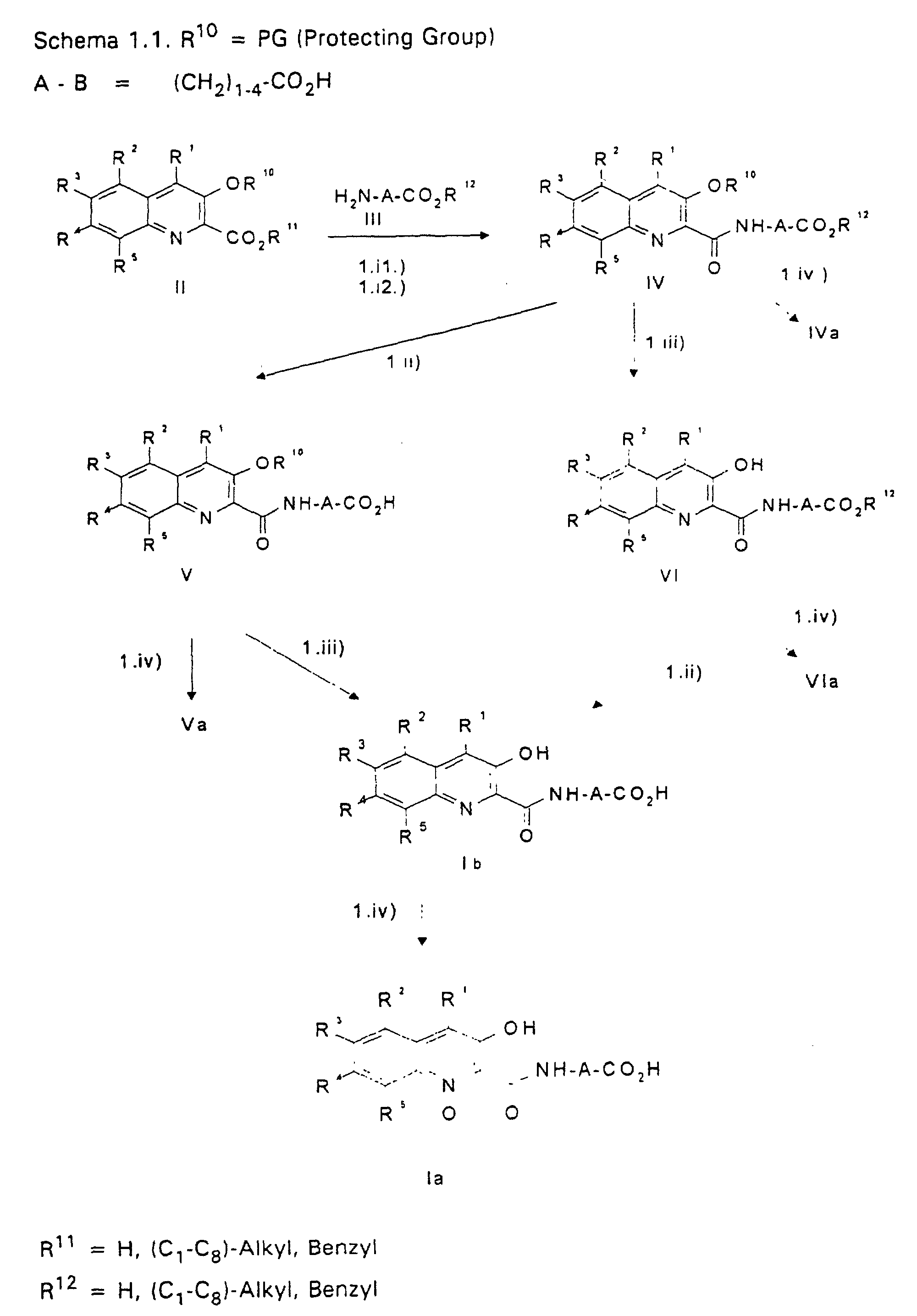

Liegt die 3-OH-Funktion in den Chinolin-2-carbonsäuren der Formel II a ohne Schutzgruppe vor, so können diese Verbindungen mit den Aminosäureresten der Formel III direkt zu den Verbindungen der Formel VI umgesetzt werden.

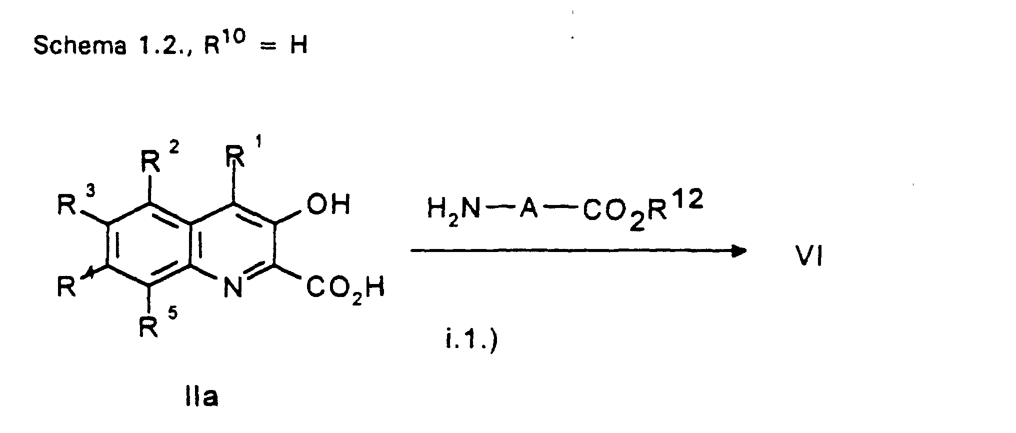

Zur Herstellung der Chinolin-N-Oxide der Formel I (= Ia) werden die Verbindungen der Formeln I, IV, V oder VI zu ihren entsprechenden N-Oxiden der Formeln Ia, IVa, Va oder VIa umgesetzt und diese analog zu Schema 1.1. weiterbehandelt.

Geeignete Verfahren zur Amidbildung (Umsetzung 1.i1.) sind die Methoden der Carboxylaktivierung und die aus der Peptidchemie bekannten Kondensationsreaktionen.

Als Reagenzien zur Carbonsäureaktivierung können die dem Fachmann bekannten Substanzen, wie Thionylchlorid, Oxalylchlorid, Pivaloylchlorid, Chlorameisensäureester-Derivate oder N,N'-Carbonyldimidazol Verwendung finden. Die aktivierten Derivate der Verbindungen der Formel II werden nach Herstellung in situ mit den Amidderivaten der Formel III umgesetzt.

Ein geeignetes Kondensationsmittel ist beispielsweise die Kombination von N,N'-Dicyclohexylcarbodiimid, 1-Hydroxy-1H-benzotriazol und N-Ethylmorpholin.

Geeignete Lösungsmittel sind Dichlormethan, Tetrachlormethan, Butylacetat, Ethylacetat, Toluol, Tetrahydrofuran, Dimethoxyethan, 1,4-Dioxan, Acetonitril, N,N-Dimethylformamid, N,N-Dimethylacetamid, Dimethylsulfoxid, Nitromethan und/oder Pyridin.

Zur Herstellung der Verbindungen der Formel II werden entsprechend substituierte Chinoline mit einer geschützten Hydroxyfunktion in 3-Position und einer oxidierbaren Gruppe G in 2-Stellung Oxidationsreaktionen unterworfen (Schema 2.1.).

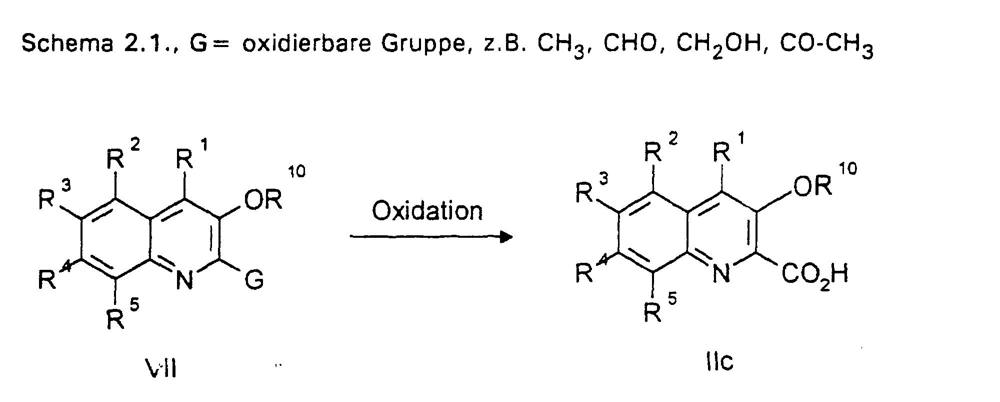

Vielfältig substituierte 3-Hydroxy-Derivate der Chinolin-2-carbonsäuren der Formel II werden entsprechend Schema 2.2 erhalten.
2.i) Substituierte 2-Nitrobenzoesäuren der Formel VIII werden nach den üblichen Methoden der Carbonsäure-Aktivierung behandelt und mit Salzen des Acetylacetons zu den Verbindungen der Formel IX umgesetzt.
2.ii) Die Verbindungen der Formel IX werden mit einer Base zu den 2-Acetyl-3-hydroxychinolinen der Formeln Xa/b cyclisiert.
2.iii) Die Verbindungen der Formel Xa werden mit einem Reagenz R¹⁰-X zu den Hydroxy-geschützten Verbindungen der Formel XI umgesetzt.
2.iv) Die Verbindungen der Formel XI werden zu den Verbindungen der Formel II oxidiert, vorzugsweise unter den Bedingungen der Haloform-Reaktion.

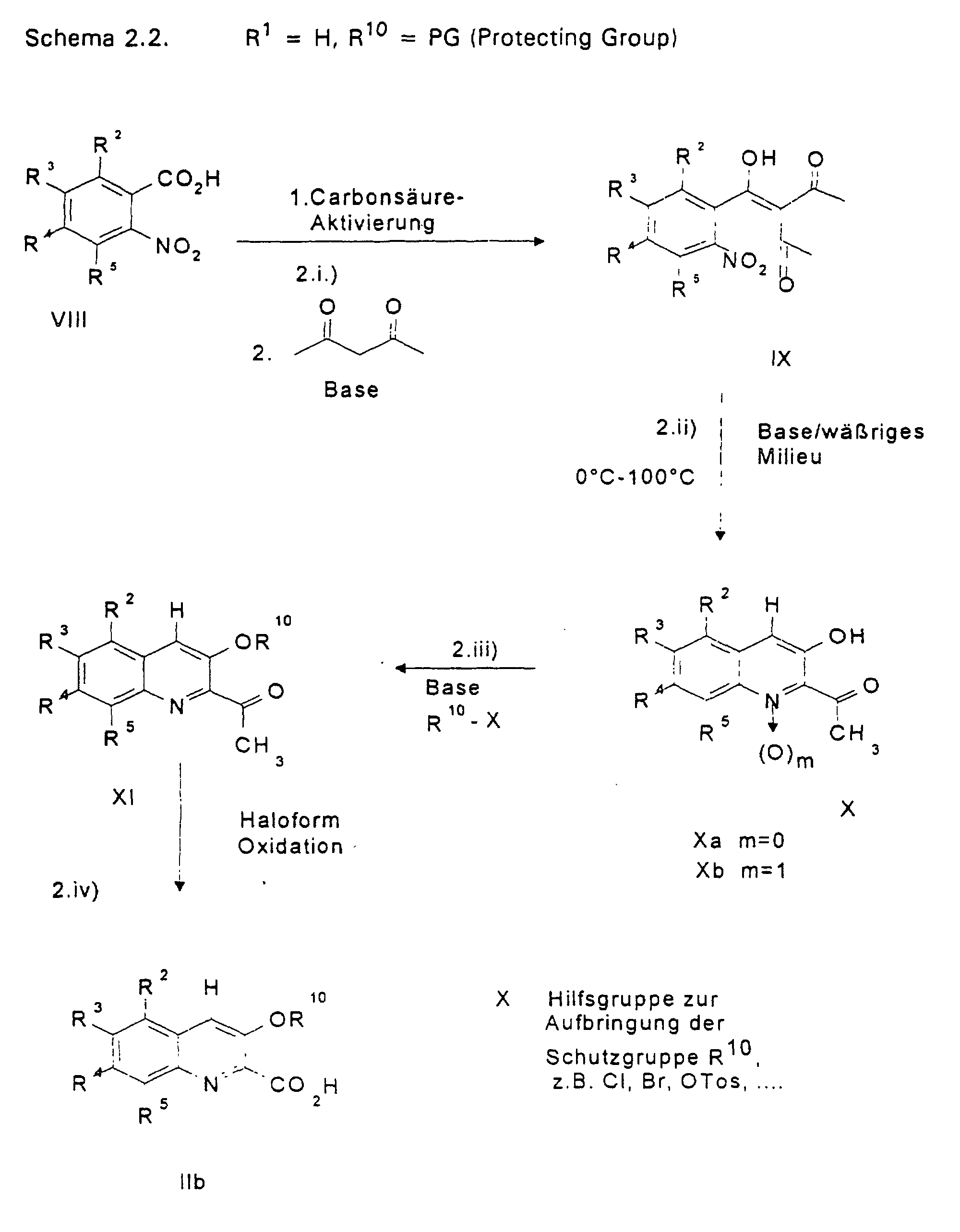

Die Herstellung von 3-(2-Nitrobenzoyl)acetylaceton (Formel IX; R²-R⁵ = H) ist bekannt, J. Prakt. Chem. 1987, 329, S. 1063.

Die Umsetzung der Verbindungen der Formel IX mit wäßriger Kaliumhydroxidlösung zu den Verbindungen der Formel Xa, in denen R², R⁴ und R⁵ Wasserstoff und R³ Wasserstoff, Methyl und Chlor ist, ist von G. Tennant et al., J. Chem. Soc. Chem. Comm. 1975, 782, bekannt.

Es wurde nun gefunden, daß die Substituenten R², R³, R⁴ und R⁵ erfindungsgemäß variiert werden können, um die beschriebene Aufgabe zu lösen.

Weiterhin wurde gefunden, daß die Verbindungen der Formel XI unter den Bedingungen der Haloform-Reaktion (Wäßriges Alkali- oder Erdalkalihydroxid/Brom) mild zu den Verbindungen der Formel II (IIb) oxidierbar sind.

2-Nitrobenzoesäuren der Formel VIII sind käuflich zu erhalten, literaturbekannt, oder können - je nach gewünschtem Substitutionsmuster - durch vielfältige synthetische Methoden hergestellt werden.

Die Verbindungen der Formeln II, IIa und IIb (Zwischenprodukte) sind neu mit folgenden Ausnahmen:
Verbindungen der Formel II, in denen - sofern R¹ bis R⁵ und R¹¹ Wasserstoff bedeuten - R¹⁰ Benzyl oder Methyl ist,
(bzw. Verbindungen der Formel IIb, in denen - sofern R² bis R⁵ Wasserstoff bedeutet - R¹⁰ Benzyl oder Methyl ist) und

Verbindungen der Formel II, in denen - sofern R¹ bis R⁵ Wasserstoff und R¹¹ Ethyl bedeuten - R¹⁰ Wasserstoff, Benzyl oder p-Toluolsulfonyl ist und

Verbindungen der Formel IIa, in denen - sofern R¹, R², R⁴ und R⁵ Wasserstoff bedeutet - R³ Wasserstoff oder Methoxy ist.

3-Benzyloxychinolin-2-carbonsäure ist aus J.Am. Chem. Soc., Vol. 115, No. 24, 1993, Seiten 11624-11625 bekannt.

3-Hydroxychinolin-2-carbonsäure und 3-Methoxychinolin-2-carbonsäuremethylester sind in J. Antibiot. 1987, 40 (7), Seiten 953-960 beschrieben, ersteres auch in Chem. Pharm. Bull. 1967, 15 (5), Seiten 663-669.

3-Hydroxy-6-methoxychinolin-2-carbonsäure ist in J. Am. Chem. Soc., Vol. 103, No.5, 1981, Seiten 1241 - 1243 beschrieben.

3-Methoxychinolin-2-carbonsäure ist aus J. Chem. Soc. 1960, Seiten 3371-3377 bekannt.

3-Hydroxychinolin-2-carbonsäureethylester und 3-(p-Toluolsulfonyloxy)chinolin-2-carbonsäureethylester sind aus J. Chem. Soc. C. 1967, 4, Seiten 256-261 bekannt.

Weiterhin sind Zwischenprodukte der Formeln Xa und Xb neu, mit folgenden Ausnahmen: Verbindungen der Formel Xa, in denen - sofern R², R⁴ und R⁵ Wasserstoff bedeuten - R³ Wasserstoff, Methyl oder Chlor ist. Diese bekannten Verbindungen der Formel Xa sind in J. Chem. Soc. Chem. Comm. 1975, Seiten 782 bis 783 beschrieben.

Die Erfindung betrifft daher auch folgende Zwischenprodukte zur Herstellung der Verbindungen der Formel I:
Zwischenprodukte der Formel II, in denen R¹ bis R⁵ die Bedeutungen haben, die für die Verbindungen der Formel I gelten,
   - R¹⁰: H oder eine HO-Schutzgruppe (Protecting Group PG) ist und
   - R¹¹: Wasserstoff, (C₁-C₈)-Alkyl und Benzyl bedeuten,
   mit der Ausnahme von Verbindungen der Formel II, in denen R¹⁰ Benzyl oder Methyl ist, sofern R¹ bis R⁵ und R¹¹ Wasserstoff bedeuten, und solchen Verbindungen aus Formel II, in denen R¹⁰ Wasserstoff, Benzyl oder p-Toluolsulfonyl ist, sofern R¹ bis R⁵ Wasserstoff und R¹¹ Ethyl bedeuten.
Zwischenprodukte der Formel Xa, in denen R² bis R⁵ die Bedeutungen haben, die für die Verbindungen der Formel I gelten,
mit der Ausnahme von Verbindungen der Formel Xa, in denen R³ Wasserstoff, Methyl oder Chlor ist, sofern R², R⁴ und R⁵ Wasserstoff bedeuten.

Alkoxy- und Benzyloxy-substituierte Chinolinsysteme der vorliegenden Erfindung werden durch Alkylierung von phenolischen OH-Gruppe(n) von 2-Nitrobenzoesäuren oder deren Derivaten, in denen nach Alkylierung die Carboxylatgruppe durch Oxidation von Methyl-, Acetyl-, Hydroxymethyl- oder Aldehydgruppen eingeführt oder durch Verseifung einer Estergruppe erhalten wird, hergestellt, vgl. z.B. Schema 3.1.

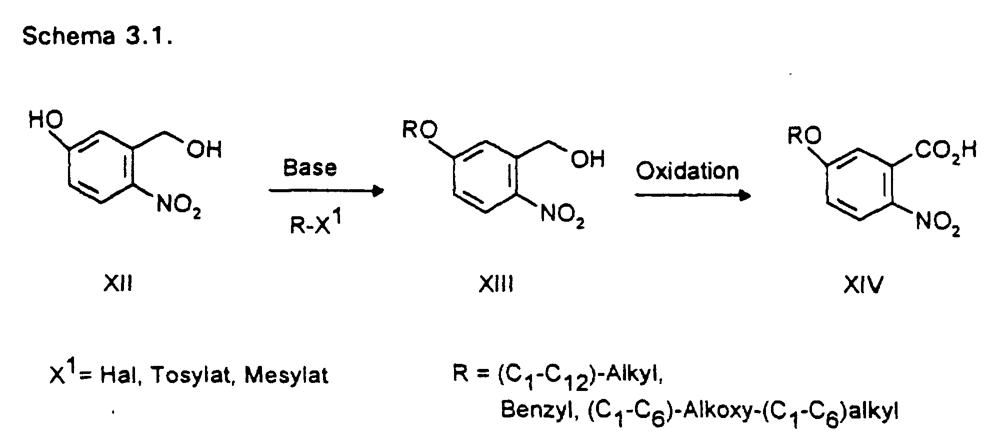

6-Aryloxychinolin- und 6-Arylsulfonyl-Derivate werden durch nucleophile aromatische Substitution an 4-Halo-2-nitrotoluolen XV und nachfolgende Oxidation zu den enstprechenden 5-Aryloxy(5-Arylsulfonyl)-2-nitrobenzoesäuren XVII zugänglich, vgl. Schema 3.2..

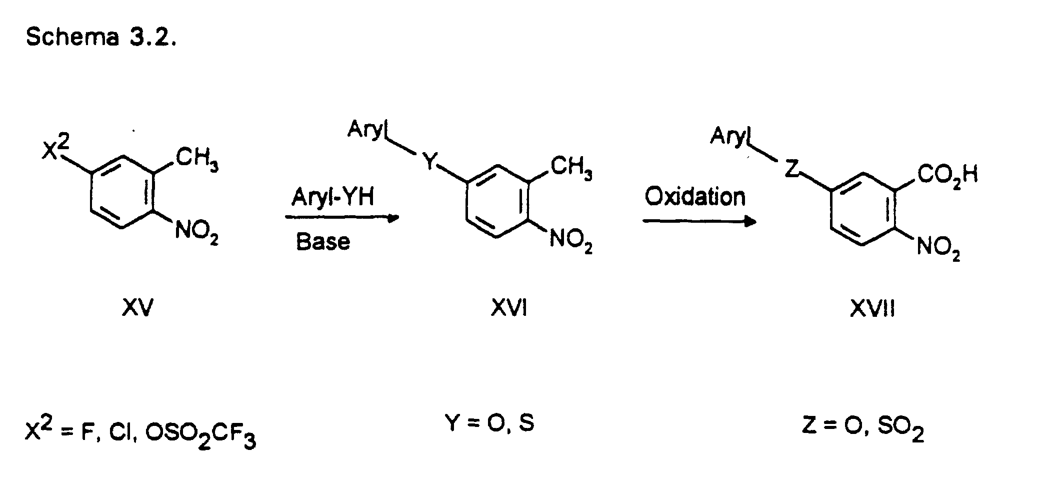

Eine weitere Möglichkeit bietet die Nitrierung von entsprechend substituierten Toluolen, Benzoesäuren, Benzaldehyden oder Acetophenonen. Bevorzugt wird diese Methode angewandt, wenn die vorhandenen Substituenten ausschließlich oder vorwiegend die Nitrierung in 2-Position lenken, vgl. z.B. Schema 3.3..

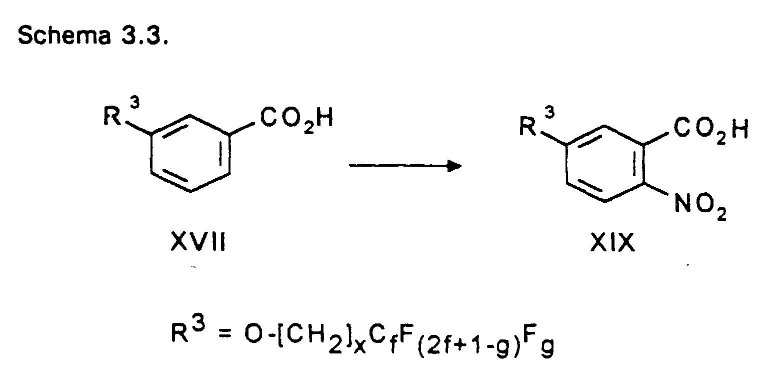

Die Verbindungen der Formeln XIII und XVI wurden mit einem Oxidationsmittel, vorzugsweise mit KMnO₄ in wäßrigem alkalischen Milieu, zu den 2-Nitrobenzoesäurederivaten umgesetzt.

Die Verbindungen der Formel I sind Inhibitoren der Prolyl-4-hydroxylase. Die Hemmung dieses Enzyms wurde, wie von Kaule und Günzler in Annal. Biochem. 184, 291 bis 297 (1990) beschrieben, bestimmt.

Die erfindungsgemäßen Verbindungen der Formel I besitzen weiterhin wertvolle pharmakologische Eigenschaften und zeigen insbesondere antifibrotische Wirksamkeit.

Die antifibrotische Wirkung kann im Modell der Tetrachlorkohlenstoffinduzierten Leberfibrose bestimmt werden. Dazu werden Ratten mit CCl₄ (1 ml/kg) - gelöst in Olivenöl - zweimal wöchentlich behandelt. Die Prüfsubstanz wird täglich, gegebenenfalls sogar zweimal täglich per os oder intraperitoneal - gelöst in einem geeigneten verträglichen Lösungsmittel - verabreicht. Das Ausmaß der Leberfibrose wird histologisch bestimmt und der Anteil Kollagen in der Leber per Hydroxyprolinbestimmung - wie bei Kivirikko et al. (Anal. Biochem. 19, 249 f. (1967)) beschrieben - analysiert. Die Aktivität der Fibrogenese kann durch radioimmunologische Bestimmung von Kollagenfragmenten und Prokollagenpeptiden im Serum bestimmt werden. Die erfindungsgemäßen Verbindungen sind in diesem Modell in Konzentration 1 bis 100 mg/kg wirksam.

Die Aktivität der Fibrogenese kann durch radioimmunologische Bestimmung des N-terminalen Propeptids des Kollagen Typ-III oder der N- bzw. C-terminalen Quervernetzungsdomäne des Kollagen-Typ-IV (7s-Kollagen bzw. Typ-IV-Kollagen NC₁) im Serum bestimmt werden.

Zu diesem Zweck wurden die Hydroxyprolin-, Prokollagen-III-Peptid-, 7s-Kollagen- und Typ-IV-Kollagen-NC-Konzentrationen in der Leber von
a) unbehandelten Ratten (Kontrolle)
b) Ratten, denen Tetrachlorkohlenstoff verabreicht wurden (CCl₄-Kontrolle)
c) Ratten, denen zunächst CCl₄ und anschließend eine erfindungsgemäße Verbindung verabreicht wurde
gemessen (diese Testmethode wird beschrieben von Rouiller, C., experimental toxic injury of the liver; in The Liver, C. Rouiller, Vol. 2, 5. 335 bis 476, New York, Academic Press, 1964).

Weiterhin kann eine Wirksamkeit der erfindungsgemäßen Verbindungen in folgenden Systemen nachgewiesen werden.

### Hemmung der hepatischen Prolyl-4-hydroxylase in vivo:

Dieses Modell dient zum Nachweis der akuten Hemmung der Prolyl-4-hydroxylase in vivo. Dazu werden Ratten beiderlei Geschlechts (gesund bzw. mit induzierter Leberfibrose) die Prüfusubstanz bzw. das entsprechende Vehikel appliziert (intraperitoneal, intravenös, per os) und nach Substanzgabe ¹⁴C-L-Prolin (250 µCi/kg Körpergewicht) intraperitoneal verabreicht. Danach erfolgt erneut eine intraperitoneale Applikation von ¹⁴C-L-Prolin (250 µCi/kg Körpergewicht). Schließlich werden die Tiere unter Pentobarbitalnarkose entblutet und die Leber entnommen. Die Aufreinigung des hepatischen Kollagens durch Pepsinverdau und fraktionierte Ammoniumsulfatfällung erfolgte entsprechend publizierten Protokollen (Ref. 1, 2). Das gereinigte Leberkollagen wurde hydrolysiert und der Gehalt an ¹⁴C-Hydroxyprolin und ¹⁴C-Prolin durch Aminosäureanalyse mittels Ionenaustauschchromatografie bestimmt. Eine Hemmung der Prolyl-4-hydroxylase ergibt sich aus einer Absenkung des Quotienten ¹⁴C-Hydroxyprolin/[¹⁴C-Hydroxyprolin + ¹⁴C-Prolin]. Als Referenzsubstanz wird 2,2'-Dipyridyl verwendet. (Ref. 1: Chojkier, M. 1986. Hepatocyte collagen production in vivo in normal rats. J. Clin. Invest. 78: 333-339 und Ref. 2: Ogata I., et al. 1991. Minor contribution of hepatocytes to collagen production in normal and early fibrotic livers. Hepatology 14: 361-367).

### Hemmung der Prolyl-4-hydroxylase in Zellkulturen:

Für die Testung von Prolyl-4-hydroxylasehemmstoffen in Zellkulturen werden folgende Zelltypen verwendet:
Normale humane Hautfibrolasten (Normal human fibrolasts, NHDF), Rattenleber-Epithelzellen (rat liver epithelial cells, Ref. 1) und primäre Fettspeicherzellen aus der Rattenleber (fat storing cells, Ref. 2). Dazu werden die Zellen in Gegenwart von Hemmstoffen kultiviert. Gleichzeitig wird das in dieser Zeit neu synthetisierte Kollagen durch 4-³H-L-Prolin und ¹⁴C-Prolin metabolisch markiert. Der Einfluß der Testsubstanzen auf den Hydroxylierungsgrad des Kollagens wird anschließend entsprechend der Methode von Chojkier et al (Ref. 3) bestimmt. Als Referenzsubstanz wird 2,2'-Dipyridyl eingesetzt. (1.: Schrode, W., Mecke, D., Gebhard, R. 1990. Induction of glutamine synthetase in periportal hepatocytes by co-cultivation with a liver epithelial cell line. Eur. J. Cell. Biol. 53: 35-41, 2. Blomhoff, R., Berg T. 1990. Isolation and cultivation of rat liver stellate cells. Methods Enzymol. 190: 59-71 und 3.: Chojkier, M. Peterkofsky, B. Bateman,, J. 1980. A new method for determining the extent of proline hydroxylation by measuring changes in the ration of [4-³H]:[¹⁴C] proline in collagenase digests. Anal. Biochem. 108: 385-393).

Die Verbindungen der Formel I können als Medikamente in Form von pharmazeutischer Präparate Verwendung finden, welche sie gegebenenfalls mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,1 bis 25 mg/kg/Tag, vorzugsweise 1 bis 5 mg/kg/Tag oder parenteral in Dosen von 0,01 bis 5 mg/kg/Tag, vorzugsweise 0,01 bis 2,5 mg/kg/Tag, insbesondere 0,5 bis 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Unter den im folgenden beschriebenen Beispielen werden die erfindungsgemäßen Verbindungen der Formel I als substituierte Chinolin-2-carbonsäure-N-(carboxymethyl)amide (Chinolin-2-carbonsäure-glycinamide) verstanden. Die Bezeichnung als substituierte N-((Chinolin-2-yl)-carbonyl)glycine wird parallel verwandt.

### Beispiel 1 (nicht erfindungsgemäß)

### 3-Hydroxychinolin-2-carbonsäure-N-(carboxymethyl)amid

a) 3-(2-Nitrobenzoyl)acetylaceton wurde aus Acetylaceton und 2-Nitrobenzoylchorid erhalten, Fp. 69°C; vgl. J. Prakt. Chem. 1987, 329, S. 1063, 29 % Ausbeute.
b) 2-Acetyl-3-hydroxychinolin wurde aus dem Produkt a) unter basischen Bedingungen (KOH/Wasser, Smiles-Umlagerung) erhalten, Fp. 105°C; vgl. J. Chem. Soc. Chem. Comm. 1975, 782; 53 % Ausbeute.
c) 2-Acetyl-3-benzyloxychinolin wurde aus dem Produkt b) mit Benzylbromid (Pottasche/Aceton) erhalten, 52 % Ausbeute,
   ¹H-NMR (CDCl₃): δ = 2,89 (s, 3 H), 5,25 (s, 2 H), 7,38 (m, 3 H), 7,58 (m, 5 H), 7,70 (m, 1 H), 8,08 (m, 1 H).
d) 3-Benzyloxychinolin-2-carbonsäure wurde aus dem Produkt c) mit Kaliumhypochlorit (Dioxan/Wasser) erhalten, öliges Rohprodukt, 47 % Ausbeute,
   ¹H-NMR (CDCl₃): δ = 5,40 (s, 2 H), 7,40 (m, 3 H), 7,63 (m, 4 H), 7,75 (m, 2 H), 8,07 (m, 1 H).
e) 3-Benzyloxychinolin-2-carbonsäure-N-((benzyloxycarbonyl)methyl)amid wurde aus dem Produkt d) mit Triethylamin/Chlorameisensäureethylester (gemischte Anhydrid-Methode) und Glycinbenzylester-Tosylat erhalten, öliges Rohprodukt, 64 % Ausbeute,
   ¹H-NMR (CDCl₃): δ = 4,40 (d, 2 H), 5,25 (s, 2 H), 5,35 (s, 2 H), 7,10 bis 7,75 (m, 14 H), 8,10 (m, 1 H), 7,28 (t, 1 H).
f) Die Titelverbindung wurde erhalten, indem das Produkt e) in Methanol mit Pd/C (10 %) in der Schüttelente hydriert wurde, Fp. 191°C (aus wäßriger Salzsäure), 40 % Ausbeute.

### Beispiel 2

### N-((3-Hydroxy-6-methoxychinolin-2-yl)carbonyl)glycin

a) 3-(5-Methoxy-2-nitrobenzoyl) acetylaceton
   Aus 19,75 g 5-Methoxy-2-nitrobenzoesäure wurden analog Beispiel 4c) mit Oxalylchlorid und Mg-Acetylacetonid 28 g Rohprodukt erhalten.
b) 2-Acetyl-3-hydroxy-6-methoxychinolin
   28 g (0,1 Mol) des vorstehenden Rohprodukts wurden in 250 ml 20 %iger wäßriger KOH-Lösung 30 min unter Rückfluß erhitzt. Man arbeitete wie unter Beispiel 4d) auf, chromatographierte das Rohprodukt mit n-Heptan/Ethylacetat (1:1) an Kieselgel und brachte aus entsprechenden Fraktionen mit Diisopropylether 5,1 g Produkt zur Kristallisation, Fp. 124-126°C.
c) 2-Acetyl-3-benzyloxy-6-methoxychinolin, aus vorstehender Verbindung durch Alkylierung mit Benzylbromid, Fp. 100-102°C (aus Diisopropylether).
d) 3-Benzyloxy-6-methoxychinolin-2-carbonsäure
   1,2 g (4mMol) der vorstehenden Acetylverbindung wurden in 5 ml 1,4-Dioxan gelöst zu einer Lösung von 1,6 g NaOH (40 mMol) in 8 ml Wasser; die bei 5-10°C mit 0,6 ml (12 mMol) Brom versetzt worden war, hinzugetropft. Nach Versetzen mit wenig Diethylether, Abtrennen der organischen Phase wurde die alkalische Lösung mit einer Lösung von 0,4 g Natriumdisulfit in 6 ml Wasser versetzt, im Vakuum eingeengt, der Rückstand in 8 ml Wasser aufgenommen, mit konz. HCl unter Kühlung auf pH 2 gestellt und das kristallisierte Produkt abgesaugt und getrocknet, 0,83 g, Fp. 272-275°C (sintern 90°C).
e) N-((3-Benzyloxy-6-methoxychinolin-2-yl)carbonyl)glycin-(1-butyl)ester
   0,83 g (2,7 mMol) der vorstehenden Carbonsäure wurden in 200 ml wasserfreiem Dichlormethan analog Beispiel 4 g) mit 0,82 g (2,7 mMol) Glycinbutylester-Tosylat, 1,1 ml (8,1 mMol) NEM, 0,405 g (3mMol) HOBT und 1,14 g (2,7 mMol) CMC umgesetzt. Das Rohprodukt wurde mit Ethylacetat an Kieselgel chromatographiert. Aus entsprechenden Fraktionen wurden mit Diisopropylether 0,8 g Produkt zur Kristallisation gebracht, Fp. 90-92°C.
f) N-((3-Benzyloxy-6-methoxychinolin-2-yl)carbonyl)glycin
   0,5 g (1,2mMol) des vorstehenden Esters wurden in 1,5 N methanolischer Natronlauge verseift, 0,45 g Produkt, Fp. 118°C (sintern bei 105°C, aus wäßriger Salzsäure).
g) Die Titelverbindung wurde erhalten, indem 0,4 g (1,1 mMol) der obigen Benzylverbindung in Methanol/Tretrahydrofuran (1:1) mit Pd/C (10 %) in der Schüttelente hydriert wurden. Nach Absaugen des Katalysators, Einengen im Vakuum brachte man den Rückstand mit Diethylether zur Kristallisation, 0,16 g, Fp. 258-260°C.

### Beispiel 3

### N-((6-( 1-Hexyloxy)-3-hydroxychinolin-2-yl)carbonyl)glycin

### Beispiel 4

### N-((6-(1-Butyloxy)-3-hydroxychinolin-2-yl)carbonyl)glycin

a) 5-(1-Butyloxy)-2-nitrobenzylalkohol
   5,1 g (30 mMol) 5-Hydroxy-2-nitrobenzylalkohol wurden in 25 ml N,N-dimethylacetamid mit 2,5 g (18 mMol) Kaliumcarbonat 30 min bei 70°-80°C gerührt. Nach Abkühlung auf 20°C wurden 3,9 ml (36 mMol) 1-Butylbromid zugetropft und 2 h bei 90°C gerührt. Nach Abkühlen wurde das Reaktionsgemisch im Vakuum eingeengt, der Rückstand mit Wasser behandelt und mit 2n HCl auf pH 7 gebracht. Das erhaltene Öl kristallisiert nach ca. 30 min; das Produkt wurde abgesaugt, mit Wasser gewaschen und getrocknet; 6 g, Fp. 47-49°C.
b) 5-(1 -Butyloxy)-2-nitrobenzoesäure
   0,5 g (10 mMol) Kaliumhydroxid wurden in 40 ml Wasser gelöst, bei 20°C unter Rühren mit 2,2 g (10 mMol) des obigen Alkohols versetzt, auf 70-80°C erwärmt und portionsweise 3,16 g (20 mMol) Kaliumpermanganat zugegeben. Nachdem 30 min bei 60-70°C gerührt wurde, saugte man heiß ab, wusch den Filterrückstand mit heißem Wasser, engte das Filtrat auf 20 ml ein und versetzte mit halbkonz. Salzsäure bis zu einem pH-Wert von 1-2. Das sich abscheidende Öl kristallisierte nach 1 h. Das Produkt wurde abgesaugt und mit Wasser gewaschen, 2 g, Fp. 82-84°C.
c) 3-(5-(1-Butyloxy)-2-nitrobenzoyl)acetylaceton
   1. 5-(1-Butyloxy)-2-nitrobenzoylchlorid
      12 g (50 mMol) der obigen Benzoesäure wurden in 90 ml wasserfreiem Tetrahydrofuran bei 5°C mit 3 Tropfen N,N-Dimethylformamid und tropfenweise mit 7 ml Oxalylchlorid (in 12 ml Tetrahydrofuran) versetzt.
      Man ließ auf Raumtemperatur erwärmen, rührte 1 h, engte im Vakuum ein und nahm den Rückstand in 25 ml wasserfreiem Toluol auf.
   2. Magnesium-acetylacetonid
      1,23 g (50 mMol) Magnesium-Späne wurden in 20 ml wasserfreiem Ethanol, 2,5 ml Xylol mit einem Jodkristall 8 h rückfließend erhitzt. Sodann wurde im Vakuum eingeengt, der Rückstand in 100 ml wasserfreiem Toluol aufgenommen und bei 20°C tropfenweise mit 5,2 ml (50 mMol) frisch destilliertem Acetylaceton versetzt und 30 min gerührt.
      Diese Lösung des Magnesium-acetylacetonids in Toluol wurde auf 0°C gekühlt und unter Rühren tropfenweise mit der unter 1. hergestellen Lösung des Säurechlorids versetzt. Man rührte 1 h bei 0°C, ließ über Nacht bei 20°C stehen, versetzte mit 200 ml Eiswasser und konz. Salzsäure auf pH 1, rührte 30 min, wusch die organische Phase mit Wasser, trocknete und erhielt nach dem Einengen im Vakuum 14 g öliges Rohprodukt.
d) 2-Acetyl-6-(1-butyloxy)-3-hydroxychinolin
   14 g (43,6 mMol) des vorstehenden Produktes wurden in 150 ml 20 %iger wäßriger KOH-Lösung 30 min rückfließend erhitzt. Nach dem Abkühlen wurde unter Rühren mit halbkonz. Salzsäure auf pH 1-2 gebracht, dreimal mit Dichlormethan extrahiert, die organische Phase getrocknet, im Vakuum eingeengt und der Rückstand mit n-Heptan/Ethylacetat (3:1) an Kieselgel chromatographiert. Aus entsprechenden Fraktionen wurden 2,5 g Produkt mit Petrolether zur Kristallisation gebracht, Fp. 71-73°C.
e) 2-Acetyl-3-benzyloxy-6-(1-butyloxy)chinolin
   2,5 g (ca. 10 mMol) des vorstehenden Produkts wurden in 30 ml N,N-Dimethylacetamid mit 1,38 g (10 mmol) Kaliumcarbonat und 1,2 ml (10 mMol) Benzylbromid umgesetzt (1 h bei 75-80°C). Man erhielt 2,2 g Produkt, Fp. 91-93°C (aus Diisopropylether).
f) 3-Benzyloxy-6-(1-butyloxy)chinolin-2-carbonsäure (Haloform-Oxidation)
   Eine Lösung von 1,2 g (30 mMol) NaOH in 6 ml Wasser wurde bei 5-10°C unter Rühren mit 0,45 ml (9 mMol) Brom versetzt. Sodann tropfte man eine Lösung von 1,05 g (3 mMol) der vorstehenden Acetylverbindung, gelöst in 5 ml 1,4-Dioxan, zu. Es wurde noch 1 h bei 20°C gerührt, mit 5 ml Diethylether extrahiert, die organische Phase eingeengt, der Rückstand mit 2n wäßriger HCl behandelt, abdekantiert, der Rückstand in Ethylacetat gelöst, mit Magnesiumsulfat getrocknet, eingeengt und der Rückstand mit Diisopropylether zur Kristallisation gebracht. Man erhielt 0,8 g, Fp. 108-110°C.
g) N-((3-Benzyloxy-6-(1-butyloxy)chinolin-2-yl)-carbonyl)glycin-(1-butyl)ester
   0,8 g (2,28 mMol) der vorstehenden Chinolin-2-carbonsäure wurden in 200 ml wasserfreiem Dichlormethan gelöst und nacheinander mit 0,74 g (2,43 mMol) Glycin-(1-butyl)ester-Tosylat, 1,1 ml (8,1 mMol) N-Ethylmorpholin (NEM), 0,405 g (3 mMol) 1-Hydroxybenztriazol (HOBT), und 1,0 g (2,43 mMol) N-Cyclohexyl-N'-(2-morpholinoethyl)carbodiimid-methyl-p-toluolsulfonat (CMC) versetzt und 30 h bei 20°C gerührt.
   Die Reaktionslösung wurde sodann mit 2n wäßriger HCl geschüttelt, ein Niederschlag mit verdünnter NaOH versetzt und mit Diethylether extrahiert, die vereinten organischen Phasen mit wäßriger Na-bicarbonat-Lösung extrahiert, getrocknet, eingeengt und der Rückstand mit n-Heptan/Ethylacetat (2:1) an Kieselgel chromatographiert. Nachdem entsprechende Fraktionen eingeengt und mit Diisopropylether behandelt wurden, erhielt man 0,55 g Produkt, Fp. 99-101°C.
h) Die Titelverbindung wurde erhalten, indem 0,5 g des vorstehenden Glycinesters mit 50 ml 1 n methanolischer NaOH bei 20°C verseift wurden. Man engte im Vakuum ein, versetzte den Rückstand mit Wasser, extrahierte mit Diethylether, säuerte die wäßrige, ölige Phase nach Zusatz von Tetrahydrofuran unter Kühlung an, engte im Vakuum ein, extrahierte die wäßrige Phase mit Dichlormethan, trocknete, engte ein und erhielt 0,46 g öliges Produkt. 0,3 g des erhaltenen Glycins wurden in 75 ml Tetrahydrofuran gelöst, mit Pd/C (10 %) versetzt und in der Schüttelente hydriert. Der Katalysator wurde abgesaugt, das Filtrat eingeengt und der Rückstand mit Diisopropylether zur Kristallisation gebracht. Man erhielt 140 mg der Titelverbindung als farblos kristalline Substanz, Fp. 180-182°C.

### Beispiel 5

### N-((6-Ethyloxy-3-hydroxychinolin-2-yl)carbonyl)glycin

### Beispiel 6

### N-((3-Hydroxy-6-(1-octyloxy)chinolin-2-yl)carbonyl)glycin

Die Titelverbindung wurde analog zu Beispiel 4 erhalten:
a) 2-Nitro-5-(1-octyloxy)benzylalkohol Das nach Behandeln mit 1n Salzsäure erhaltene Öl kristallisierte langsam durch, Fp. 55-57°C.
b) 2-Nitro-5-(1-octyloxy)benzoesäure, öliges Rohprodukt.
c) 3-(2-Nitro-5-(1-octyloxy)benzoyl)acetylaceton; öliges Produkt (nach Chromatographie mit n-Heptan/Ethylacetat (3:1) an Kieselgel).
d) 2-Acetyl-3-hydroxy-6-(1-octyloxy)chinolin, öliges Produkt, das beim Stehen erstarrt.
e) 2-Acetyl-3-benzyloxy-6-(1-octyloxy)chinolin; öliges Produkt, das beim Stehen durchkristallisierte.
f) 3-Benzyloxy-6-(1-octyloxy)chinolin-2-carbonsäure; Fp. 145°C (unter Zers., aus wäßriger Salzsäure/Tetrahydrofuran).
g) N-((3-Benzyloxy-6-(1-octyloxy)chinolin-2-yl)carbonyl)glycinbenzylester; Fp. 93-95°C (aus Petrolether).
h) Die Titelverbindung vurde erhalten, indem die vorstehende Benzylverbindung mit Pd/C hydriert wurde; Fp. 153-155°C (aus Petrolether).

### Beispiel 7

### N-((6-(1-Decyloxy)-3-hydroxychinolin-2-yl)carbonyl)glycin

### Beispiel 8

### N-((3-Hydroxy-6-((2,2,2-trifluorethyl)oxy)chinolin-2-yl)carbonyl)glycin-Hydrochlorid

0,5 g (1,3 mMol) N-((7-Chlor-3-hydroxy-6-(2,2,2-trifluorethyloxy)chinolin-2-yl)carbonyl)glycin (Titelverbindung aus Beispiel 31) wurden in 26 ml methanolischer Ameisensäure (4,4%) gelöst und bei 20°C mit weiteren 26 ml methanolischer Ameisensäure versetzt. Nach Zugabe von 0,5 g Pd/C (10%) wurde 1 h bei 20°C gerührt, vom Katalysator abfiltriert, das Filtrat eingeengt und der Rückstand mit Diisopropylether zur Kristallisation gebracht. Man erhielt 250 mg Produkt, Fp. >300°C (sintern bei 207°C).

### Beispiel 9

### N-((3-Hydroxy-6-((2,2,3,3,3-pentafluorpropyl)oxy)chinolin-2-yl)carbonyl)glycin

### Beispiel 10

### N-((6-((2,2,3,3,4,4,4-Heptafluorbutyl)oxy)-3-hydroxychinolin-2-yl)carbonyl)glycin

### Beispiel 11

### N-((6-Chlor-3-hydroxychinolin-2-yl)carbonyl)glycin

a) 2-Acetyl-6-chlor-3-hydroxychinolin, vgl. J. Chem. Soc. Chem. Comm. 1975, 782.
b) 2-Acetyl-3-benzyloxy-6-chlorchinolin
   5,0 g (22,5 mMol) des vorstehenden Chlorchinolins wurden in 50 ml N,N-Dirnethylacetamid gelöst, 30 min mit 3,4 g (25 mMol) Kaliumcarbonat bei 70-80°C gerührt, bei 20°C 3,1 ml (25 mMol) Benzylbromid zugetropft und 2 h bei 90°C gerührt. Nach Aufarbeitung wurde mit n-Heptan/Ethylacetat (4:1) an Kieselgel chromatographiert und aus entsprechenden Fraktionen 4,2 g Produkt mit Diisopropylether zur Kristallisation gebracht, Fp. 107-110°C.
c) 3-Benzyloxy-6-chlorchinolin-2-carbonsäure
   2,1 g (6,7 mMol) der vorstehenden Acetylverbindung, gelöst in 7 ml 1,4-Dioxan, wurden bei 0°C zu einer Lösung von 1 ml (20 mMol) Brom in wäßriger Natronlauge (aus 2,7 g (6,7 mMol) in 13,5 ml Wasser) hinzugetropft. Nach Aufarbeitung erhielt man 1,2 g Produkt, Fp. 90-93°C (sintern ab 80°C, aus wäßriger Salzsäure).
d) N-((3-Benzyloxy-6-chlorchinolin-2-yl)carbonyl)glycin-(1-butyl)ester
   Das Produkt wurde analog Beispiel 4g) aus 1,1 g (3,3 mMol) der vorstehenden Chinolincarbonsäure und den Kupplungsreagenzien NEM, HOBT, CMC und Glycin-(1-butyl)ester-Tosylat erhalten, 0,75 g, Fp. 121-123°C (aus Diisopropylether).
e) Die Titelverbindung wurde erhalten, indem 0,3 g (0,75 mMol) des vorstehenden Glycinesters in 25 ml 1 n methanolischer Natronlauge 1 h bei 20°C verseift wurden. Man isolierte 0,3 g des harzigen Na-Salzes, das mit 12,5 ml 48 %iger wäßriger HBr versetzt und 1 h bei 80-85°C Badtemperatur gerührt wurde. Nach Einengen im Vakuum wurde der kristalline Rückstand mit Tetrahydrofuran extrahiert, im Vakuum eingeengt und der Rückstand mit Diisopropylether zur Kristallisation gebracht. Man erhielt 0,16 g der Titelverbindung, Fp. 224°C (unter Zers.).

### Beispiel 12

### N-((6-Brom-3-hydroxychinolin-2-yl)carbonyl)glycin

### Beispiel 13

### N-((3-Hydroxy-6-(phenylsulfonyl)chinolin-2-yl)carbonyl)glycin

a) 2-Nitro-5-(phenylsulfonyl)toluol
   In 150 ml wasserfreiem Ethanol wurden unter Rühren und Erwärmen 4 g (100 mMol) NaOH gelöst, bei 20°C 10,3 ml (100 mMol) Thiophenol zugetropft und nach 10 min. 15,5 g (12,2 ml; 100 mMol) 5-Fluor-2-nitrotoluol, gelöst in 20 ml Ethanol zugetropft. Nach 30 min bei 20°C wurde 30 min zum Rückfluß erhitzt und heiß abfiltriert. Nach Anreiben setzte die Kristallisation ein. Nach Zugabe von Wasser wurde abgesaugt und getrocknet; 19 g 2-Nitro-5-phenylthiotoluol, Fp. 70-71 °C. Anschließend wurde in Dichlormethan mit m-Chlorperbenzoesäure oxidiert; 20,5 g Produkt, Fp. 108-110°C (aus Petrolether).
b) 2-Nitro-5-(phenylsulfonyl)benzoesäure
   15 g (55 mMol) 2-Nitro-5-phenylsulfonyltoluol wurden in 210 ml Pyridin/Wasser (1:2) suspendiert und unter Rückfluß jeweils bis zur Entfärbung portionsweise mit 79 g (500 mMol) Kaliumpermanganat versetzt. Dann wurde heiß abgesaugt, dreimal mit heißem Wasser nachgewaschen. Im Filtrat ausgefallenes Ausgangsmaterial (2,8 g) wurden zurückgewonnen. Das zweite Filtrat wurde eingeengt, mit 200 ml Wasser versetzt, mit Diethylether extrahiert und die wäßrige Phase unter Kühlung mit halbkonz. wäßriger HCl auf pH 1 gebracht. Das ausgefallene Produkt wurde abgesaugt und getrocknet; 9 g, Fp. 210-212°C.
c) 3-(2-Nitro-5-(phenylsulfonyl)benzoyl)acetylaceton
   Aus 9 g (30 mMol) der vorstehenden Substanz wurden analog zu Beispiel 4 c) mit Oxalylchlorid und Magnesiumacetylacetonid 9,4 g Produkt erhalten, Fp. 143-145°C (sintern bei 140°C, aus Diisopropylether).
d) 2-Acetyl-3-hydroxy-6-(phenylsulfonyl)chinolin
   9,5 g der vorstehenden Verbindung wurden unter Rühren bei 20°C in 95 ml wäßrige Kaliumhydroxid-Lösung (20%) eingetragen, wobei nach 45 min eine klare Lösung entstand. Nach 90 min wurde unter Kühlung mit halbkonz. wäßriger HCL auf pH 1 gebracht und das Rohprodukt abgesaugt. Dieses wurde mit n-Heptan/Ethylacetat (3:1) an Kieselgel chromatographiert. Aus entsprechenden Fraktionen isolierte man 1,5 g Produkt, Fp. 179-180°C.
e) 2-Acetyl-3-benzyloxy-6-(phenylsulfonyl)chinolin
   1,5 g (4,6 mMol) der vorstehenden Verbindung wurden in 50 ml Diethylketon mit 0,63 g (4,6 mMol) Kaliumcarbonat und 0,55 ml (4,6 mMol) Benzylbromid umgesetzt (3 h zum Rückfluß erhitzt). Man erhielt 2,0 g Produkt, Fp. 160-162°C (aus Wasser).
f) 3-Benzyloxy-6-(phenylsulfonyl)chinolin-2-carbonsäure
   2,0 g (4,8 mMol) der vorstehenden Acetyl-Verbindung wurden analog Beispiel 4 f) der Haloform Reaktion unterworfen. Man erhielt 1,4 g Produkt, das weiter umgesetzt wurde.
g) N-((3-Benzyloxy-6-(phenylsulfonyl)chinolin-2-yl)carbonyl)glycinbenzylester
   1,4 g (3,3 mMol) der vorstehenden Carbonsäure wurden analog Beispiel 4 g) mit Glycinbenzylester-Tosylat, N-Ethylmorpholin, 1-Hydroxy-1H-benztriazol und CMC umgesetzt. Man erhielt 1,1 g Produkt, Fp. 165-167°C (aus Diethylether).
h) Die Titelverbindung wurde erhalten, indem 1,1 g (1,9 mMol) des vorstehenden Benzylesters in Tetrahydrofuran mit Pd/C (10%) in der Schüttelente hydriert wurden. Man erhielt 0,5 g farbloses Produkt, Fp. 196-198°C (aus Diisopropylether).

### Beispiel 14

### N-((6-((4-Fluorphenyl)sulfonyl)-3-hydroxychinolin-2-yl)carbonyl)glycin

### Beispiel 15

### N-((6-Benzyloxy-3-hydroxychinolin-2-yl)carbonyl)glycin

### Beispiel 16

### N-((6-(4-Fluorbenzyloxy)-3-hydroxychinolin-2-yl)carbonyl)glycin

### Beispiel 17

### N-((7-Butyloxy-3-hydroxychinolin-2-yl)carbonyl)glycin

### Beispiel 18

### N-((7-Benzyloxy-3-hydroxychinolin-2-yl)carbonyl)glycin

### Beispiel 19

### N-((6-(cis-3-Hexenyl-1-oxy)-3-hydroxychinolin-2-yl)carbonyl)glycin

### Beispiel 20

### N-((6-(trans-3-Hexenyl-1-oxy)-3-hydroxychinolin-2-yl)carbonyl)glycin

### Beispiel 21

### N-((3-Hydroxy-6-trifluormethoxychinolin-2-yl)carbonyl)glycin

a) 2-Nitro-5-trifluormethoxybenzoesäure
   76,5 ml konz. Schwefelsäure wurden unter Kühlung und Rühren tropfenweise mit 29,7 ml Salpetersäure versetzt und anschließend 9,0 g (43,7 mMol) 3-Trifluormethoxybenzoesäure hinzugegeben. Man erwärmt 1 h auf 50-55°C, gibt die Reaktionsmischung nach Abkühlen auf Eis, saugt den Rückstand ab und erhält nach dem Trocknen 8,8 g Produkt, Fp. 90-93°C (sintern bei 85°C).
b) 3-(2-Nitro-5-trifluormethoxybenzoyl)acetylaceton
   wurde analog Beispiel 4c) aus 8,8 g (35 mMol) der vorstehenden 2-Nitro-5-trifluormethoxybenzoesäure erhalten, 13 g öliges Rohprodukt.
c) 2-Acetyl-3-hydroxy-6-trifluormethoxychinolin
   11,5 g (34,5 mMol) des vorstehenden Produkts wurden unter Rühren bei 20°C in 105 ml 20 %ige wäßrige Kaliumhydroxid-Lösung eingetragen und 30 min rückfließend erhitzt. Nach dem Abkühlen wurde mit halbkonz. wäßriger HCl auf pH 1 gebracht, abgesaugt und das Rohprodukt (11 g) mit n-Heptan/Ethylacetat an Kieselgel chromatographiert. Entsprechende Fraktionen wurden eingeengt und mit Petrolether zur Kristallisation gebracht; 5,1 g, Fp. 87-88°C. Als Nebenprodukt wurden 0,45 g des entsprechenden N-Oxids erhalten, Fp. 190-191 °C.
d) 2-Acetyl-3-benzyloxy-5-trifluormethoxychinolin
   5,0 g (18,4 mMol) der vorstehenden Verbindung wurden analog Beispiel 4e) in Diethylketon mit 2,2 ml (18,4 mMol) Benzylbromid umgesetzt; 5,6 g Produkt, Fp. 90-92°C (aus Petrolether).
e) 3-Benzyloxy-5-trifluormethoxychinolin-2-carbonsäure
   Aus 2,7 g (7,5 mMol) der vorstehenden Verbindung wurden mit 1,15 ml (22,5 mMol) Brom in Natronlauge /1,4-Dioxan 2,9 g Produkt erhalten, Fp. 286°C (unter Gasentwicklung, aus der Reaktionslösung).
f) N-((3-Benzyloxy-5-trifluormethoxychinolin-2-yl)carbonyl)glycinbenzylester
   Aus 1,8 g (5mMol) der vorstehenden Chinolin-2-carbonsäure, 1,7 g (5 mMol) Glycinbenzylester-Tosylat, 2,7 ml (20 mMol) NEM, 0,74 g (5,5 mMol) HOBT und 2,1 g (5 mMol) CMC wurden analog Beispiel 4 g) 1,35 g Produkt erhalten, Fp. 123-124°C (aus Diisopropylether).
g) Die Titelverbindung wurde erhalten, indem 1,35 g (2,65 mMol) des vorstehenden Glycinbenzylester in 100 ml Tetrahydrofuran in der Schüttelente mit Pd/C (10 %) hydriert wurden. Nach Absaugen des Katalysators, Einengen wurde der Rückstand mit Petrolether zur Kristallisation gebracht und man erhielt 0,7 g Produkt, Fp. 192-194°C.

Aus entsprechenden Vorstufen wurden die Beispiele 22, 23 und 24 analog zu Beispiel 21 erhalten.

### Beispiel 22

### N-((6-((Heptafluorpropyl)oxy)-3-hydroxychinolin-2-yl)carbonyl)glycin

### Beispiel 23

### N-((3-Hydroxy-6-((nonafluorbutyl)oxy)chinolin-2-yl)carbonyl)glycin

### Beispiel 24

### N-((3-Hydroxy-6-((pentafluorethyl)oxy)chinolin-2-yl)carbonyl)glycin

### Beispiel 25

### N-((6-(3-(Ethyloxy)-1-propyloxy)-3-hydroxychinolin-2-yl)carbonyl)glycin

a) 5-(3-Ethyloxy)-1-propyloxy)-2-nitro-benzylalkohol
   Analog Beispiel 4a) wurden 5,0 g (∼ 30 mMol) 5-Hydroxy-2-nitrobenzylalkohol mit 7,1 g (ca. 40 mMol) 3-Ethoxy-1-propylbromid (aus 8,3 g (9,2 ml, 80 mMol) 3-Ethoxy-1-propanol und 15,3 ml (160 mMol) Phosphortribromid in Dichlormethan hergestellt) umgesetzt; 6,1 g Rohprodukt.
b) 5-(3-(Ethyloxy)-1-propyloxy)-2-nitrobenzoesäure
   Analog Beispiel 4b) wurden aus 10,2 g (40 mMol) des vorstehenden Alkohols 8,1 g Produkt erhalten, Fp. 109-111 °C (aus wäßriger Salzsäure).
c) 3-(5-(3-Ethy)oxy-1-propyloxy)-2-nitrobenzoyl)acetylaceton
   Aus 10,2 g (ca. 40 mMol) der vorstehenden Benzoesäure wurden analog Beispiel 4c) nach Chromatographie mit n-Heptan/Ethylacetat (1:1) an Kieselgel 10,3 öliges Rohprodukt erhalten.
d) 2-Acetyl-6-(3-ethyloxy-1-propyloxy)-3-hydroxychinolin wurde durch Umsetzung von 10 g der vorstehenden Verbindung in 100 ml 20 %iger, wäßriger Kaliumhydroxid-Lösung (40 min, 60°C) erhalten, 2,8 g, Fp. 62-64°C (aus n-Heptan/Ethylacetat (3: 1 )).
e) 2-Acetyl-3-benzyloxy-6-(3-ethyloxy-1-propyloxy)chinolin
   2,8 g (10 mMol) der vorstehenden Verbindung wurden in 80 ml Diethylketon mit 1,4 g Kaliumcarbonat und 1,2 ml (10 mMol) Benzylbromid umgesetzt, nach Chromatographie mit n-Heptan/Ethylacetat (1:1) an Kieselgel 3,8 g öliges Produkt.
f) 3-Benzyloxy-6-(3-ethyloxy-1-propyloxy)chinolin-2-carbonsäure
   3,8 g (10 mMol) der vorstehenden Acetylverbindung wurden mit wäßriger NaOH/Brom/1,4-Dioxan oxidiert (Haloform-Reaktion). Man erhielt 3,0 g Produkt, Fp. 117-119°C (aus wäßriger Salzsäure).
g) N-((3-Benzyloxy-6-(3-ethyloxy-1-propyloxy)chinolin-2-yl)carbonyl)glycinbenzylester
   Analog den Beispielen 4g) und 21 f) wurden aus 2,3 g (6 mMol) der vorstehenden Carbonsäure in 300 ml wasserfreiem Dichlormethan mit 2,0 g (6 mMol) Glycinbenzylester-Tosylat, 3,3 ml (24 mMol) NEM, 1,0 g (7,5 mMol) HOBT und 2,7 g (6,3 mMol) CMC nach Chromatographie mit Ethylacetat an Kieselgel 2,7 g öliges Produkt erhalten.
h) Die Titelverbindung wurde erhalten, indem 2,7 g der vorstehenden Dibenzylverbindung in 100 ml Tetrahydrofuran gelöst und mit Pd/C (10 %) in der Schüttelente hydriert wurden. Dann wurde der Katalysator abgesaugt, das Filtrat im Vakuum eingeengt und der Rückstand mit Petrolether zur Kristallisation gebracht. Man erhielt 1,3 g Produkt, Fp. 133-135°C.

### Beispiel 26

### N-((3-Hydroxy-6-(2-propyloxy)chinolin-2-yl)carbonyl)glycin

a) 2-Nitro-5-(2-propyloxy)benzylalkohol
   10 g (60 mMol) 5-Hydroxy-2-nitrobenzylalkohol wurden analog Beispiel 4a) mit 7,0 ml (70 mMol) Isopropyljodid umgesetzt; nach Reinigung mit n-Heptan/Ethylacetat (3:2) an Kieselgel, 10,3 g öliges Rohprodukt.
b) 2-Nitro-5-(2-propyloxy)benzoesäure
   10,3 g (ca. 50 mMol) des vorstehenden Benzylalkohols wurden analog Beispiel 4b) oxidiert; 9,4 g Produkt, Fp. 131-133°C (aus Dichlormethan).
c) 3-(5-(2-Propyloxy)-2-nitrobenzoyl)acetylaceton
   Aus 9 g (40 mMol) des vorstehenden Produkts wurden 12,4 g Produkt gewonnen (analog Bspl. 4c)).
d) 2-Acetyl-3-hydroxy-6-(2-propyloxy)chinolin
   Analog Beispiel 4d) wurden 12,3 g (40 mMol) des vorstehenden Produkts in 150 ml 20 %iger wäßriger Kaliumhydroxid-Lösung 45 min bei 50°C umgesetzt. Man erhielt nach Chromatographie 4,3 g Produkt, Fp. 95-97°C (aus n-Heptan/Ethylacetat (3:1)).
e) 2-Acetyl-3-benzyloxy-6-(2-propyloxy)chinolin
   4,2 g (17 mMol) der vorstehenden Substanz wurden in 100 ml Diethylketon mit 1,4 g (10 mMol) Kaliumcarbonat und 2,9 g (2,0 ml, 17 mMol) Benzylbromid umgesetzt, 5,0 g Produkt, Fp. 102-104°C (aus Petrolether).
f) 3-Benzyloxy-6-(2-propyloxy)chinolin-2-carbonsäure
   3,4 g (10 mMol) der vorstehenden Acetylverbindung wurden bei 0 bis 5°C, in 10 ml 1,4-Dioxan gelöst, zu einer Lösung von 4 g NaOH und 1,6 g (30 mMol) Brom in 20 ml Wasser getropft. Nachdem ein dicker Brei entstanden war, gab man 50 ml Diethylether zu, rührte, gab dann 1 g Natriumdisulfit in 15 ml Wasser zu, saugte die Fällung ab, löste diese in Tetrahydrofuran/Wasser, säuerte mit halbkonz. wäßriger Salzsäure an, extrahierte die wäßrige Phase mit Ethylacetat, engte ein und brachte den Rückstand mit Diisopropylether zur Kristallisation. Man erhielt 3,0 g Produkt, Fp. 140°C (unter Zers.).
g) N-((3-Benzyloxy-6-(2-propyloxy)chinolin-2-yl)carbonyl)glycinbenzylester
   Analog Beispiel 25g) wurden 2,7 g (8 mMol) der vorstehenden Chinolincarbonsäure in 300 ml wasserfreiem Dichlormethan mit 2,7 g (8 mMol) Glycinbenzylester-Tosylat, 4,4 ml (32 mMol) NEM, 1,35 g (10 mMol) HOBT und 3,6 g (8,8 mMol) CMC 24 h bei 20°C umgesetzt. Nach Aufarbeitung und Chromatographie mit n-Heptan/Ethylacetat (3:2) an Kieselgel erhielt man 3,5 g öliges Produkt.
h) Die Titelverbindung wurde erhalten, indem 3,4 g (7 mMol) der vorstehenden Dibenzylverbindung in 100 ml Tetrahydrofuran mit Pd/C (10 %) in der Schüttelente hydriert wurden (260 ml Wasserstoff-Aufnahme). Man saugte den Katalysator ab, engte ein, brachte den Rückstand mit Diisopropylether zur Kristallisation. Man erhielt 1,5 g der farblosen Titelverbindung, Fp. 149-151 °C.

### Beispiel 27

### N-((3-Hydroxy-6-phenoxychinolin-2-yl)carbonyl)glycin

a) 2-Nitro-5-phenoxytoluol
   14,1 g (150 mMol) Phenol wurden unter Rühren in 120 ml wasserfreiem N,N-Dimethylacetamid gelöst, mit 20,6 g (150 mMol) fein gepulvertem Kaliumcarbonat versetzt und 30 min bei 70-80°C gerührt. Nach dem Abkühlen auf 20°C wurden 23,25 g (18,3 ml, 150 mMol) 5-Fluor-2-nitrotoluol zugetropft und 2 h auf 135-140°C erhitzt, nach dem Abkühlen im Vakuum eingeengt, der Rückstand in Eis eingetragen, das kristalline Produkt abgesaugt, mit Wasser gewaschen und getrocknet, 30 g, Fp. 43-46°C.
b) 2-Nitro-5-phenoxybenzoesäure
   (vgl. auch Liebigs Ann. 593, 113 (1955))
   13 g (56,7 mMol) der vorstehenden Substanz wurden in Pyridin/Wasser (1:2) mit 85 g Kaliumpermanganat 4 h unter Rückfluß gerührt. Es wurde heiß vom Braunstein abgesaugt, mit heißem Wasser nachgewaschen, eingeengt, der Rückstand in 250 ml gesättigter wäßriger Na-bicarbonat-Lösung aufgenommen, nicht umgesetztes Edukt abgesaugt und das Filtrat mit halbkonzentrierter HCl auf pH 1 gebracht, 4,3 g, Fp. 148-150°C.
c) 3-(2-Nitro-5-phenoxybenzoyl)acetylaceton
   Aus 15,6 g (60 mMol) der vorstehenden Benzoesäure wurden analog zu Beispiel 4 c) mit Oxalylchlorid und Magnesium-acetylacetonid 12 g öliges Rohprodukt erhalten.
d) 2-Acetyl-3-hydroxy-6-phenoxychinolin
   10 g (29,3 mMol) der vorstehenden Verbindung wurden analog Beispiel 4 d) in 100 ml 20 %iger wäßriger KOH-Lösung umgesetzt. Nach Chromatographie des Rohproduktes mit n-Heptan/Ethylacetat (3:1) an Kieselgel wurden 2,5 g Produkt erhalten, Fp. 131-133°C. Als Nebenprodukt wurden 0,55 g des entsprechenden N-Oxids erhalten, Fp. 173-175°C.
e) 2-Acetyl-3-benzyloxy-5-phenoxychinolin
   2,5 g (8,9 mMol) der vorstehenden Verbindung wurden in 80 ml Diethylketon mit 1,2 g (8,9 mMol) Kaliumcarbonat und 1,1 ml (8,9 mMol) Benzylbromid umgesetzt, 3,3 g Produkt, Fp. 116-118°C (aus Wasser, sintern bei 100°C).
f) 3-Benzyloxy-6-phenoxychinolin-2-carbonsäure
   2,2 g (5,96 mMol) der obigen Verbindung wurden analog Beispiel 4 f) (12 ml Wasser, 2,6 g NaOH (60 mMol), 0,9 ml (18 mMol) Brom, 10 ml Dioxan) der Haloform-Reaktion unterworfen, 2,1 g Produkt, Fp. 281°C-283°C.
g) N-((3-Benzyloxy-6-phenoxychinolin-2-yl)carbonyl)glycinbenzylester
   1,5 g (4 mMol) der vorstehenden Carbonsäure wurden in 250 ml wasserfreiem Dichlormethan mit 1,35 g (4 mMol) Glycinbenzylester-Tosylat, 2,2 ml (16 mMol) NEM, 0,67 g (5 mMol) HOBT und 1,8 g (4,2 mMol) CMC analog Beispiel 4 g), umgesetzt. Man erhielt 1,5 g Produkt, Fp. 143-145°C (aus Diisopropylether).
h) Die Titelverbindung wurde erhalten, indem 1,5 g des vorstehenden Glycinesters in Tetrahydrofuran mit Pd/C (10 %) in der Schüttelente hydriert wurden. Man erhielt 0,7 g der Titelverbindung, Fp. 225-227°C (aus Diisopropylether).

### Beispiel 28

### N-((3-Hydroxy-6-(3-methoxyphenoxy)chinolin-2-yl)carbonyl)glycin

Die Titelverbindung wurde analog Beispiel 27 erhalten.
a) 5-((3-Methoxyphenyl)oxy)-2-nitrotoluol
   Aus 18 g (150 mMol) 3-Methoxyphenol und 23,3 g (150 mMol) 5-Fluor-2-nitrotoluol erhielt man 35 g Produkt, Fp. 48-50°C (aus Wasser).
b) 5-(3-Methoxyphenoxy)-2-nitrobenzoesäure
   Fp. 146-151 °C (aus wäßriger Salzsäure).
c) 3-(5-((3-Methoxyphenyl)oxy)-2-nitrobenzoyl)acetylaceton nach Säulenchromatographie öliges Produkt.
d) 2-Acetyl-3-hydroxy-6-((3-methoxyphenyl)oxy)chinolin
   Aus 7,5 g (20 mMol) der vorstehenden Substanz wurden nach Behandlung mit 75 ml 20%iger KOH (90 min, 55°C) 4,9 g Produkt erhalten, Fp. 102-104°C (aus n-Heptan/Ethylacetat (3:1 )).
e) 2-Acetyl-3-benzyloxy-6-((3-methoxyphenyl)oxy)chinolin öliges Produkt nach Chromatographie an Kieselgel.
f) 3-Benzyloxy-6-((3-methoxyphenyl)oxy)chinolin-2-carbonsäure, Fp. 142°C (unter Zers., aus Diisopropylether).
g) N-((3-Benzyloxy-6-((3-methoxyphenyl)oxy)chinolin-2-yl)carbonyl)-glycinbenzylester,
   Fp. 118-120°C (aus Petrolether).
h) Die Titelverbindung wurde analog Beispiel 27 h) erhalten.
   Aus 2 g der vorstehenden Substanz erhielt man 1,1 g Produkt, Fp. 166-167°C (aus Diisopropylether).

### Beispiel 29

### N-((3-Hydroxy-6-((3-trifluormethylphenyl)oxy)chinolin-2-yl)carbonyl)glycin

Die Titelverbindung wurde analog Beispiel 27 erhalten.
a) 2-Nitro-5-((3-trifluormethylphenyl)oxy)toluol
   Aus 36,5 g ( 225 mMol) 3-Hydroxybenzotrifluorid und 27,9 g (225 mMol) 5-Fluor-2-nitrotoluol wurden 62 g öliges Rohprodukt erhalten.
b) 2-Nitro-5-((3-trifluormethylphenyl)oxy)benzoesäure, Fp. 153-155°C (aus wäßriger Salzsäure).
c) 3-(2-Nitro-5-((3-trifluormethylphenyl)oxy)benzoyl)acetylaceton
   Aus 18 g ( 55 mMol) der vorstehenden Verbindung wurden nach Chromatographie mit n-Heptan/Ethylacetat (4:1) 15 g öliges Produkt erhalten.
d) 2-Acetyl-3-hydroxy-6-((3-trifluormethylphenyl)oxy)chinolin, Fp. 97-99°C (aus Petrolether).
e) 2-Acetyl-3-benzyloxy-6-((3-trifluormethylphenyl)oxy)chinolin, nach Chromatographie öliges Produkt.
f) 3-Benzyloxy-6-((3-trifluormethylphenyl)oxy)chinolin-2-carbonsäure
   5,7 g (13 mMol) der vorstehenden Acetylverbindung wurden analog Beispiel 4 f) oxidiert. Ohne Ansäuern erhielt man aus der organischen Phase 5,7 g Produkt, Fp. 150°C (aus Petrolether, schäumen).
g) N-((3-Benzyloxy-6-((3-trifluormethylphenyl)oxy)chinolin-2-yl)carbonyl)-glycinbenzylester
   5,6 g (12,7 mMol) der vorstehenden Carbonsäure wurden analog Beispiel 4 g) mit Glycinbenzylester-Tosylat, NEM, HOBT und CMC umgesetzt; 3,5 g Produkt, Fp. 103-105°C.
h) Die Titelverbindung wurde erhalten, indem 3,4 g des obigen Benzylesters in Tetrahydrofuran mit Pd/C (10%) in der Schüttelente hydriert wurden. Man isolierte 2,2 g Produkt, Fp. 166-168°C (aus Petrolether).

### Beispiel 30

### N-((7-Chlor-3-hydroxychinolin-2-yl)carbonyl)glycln

a) 3-(4-Chlor-2-nitrobenzoyl)acetylaceton
   Aus 60,5 g (0,3 Mol) 4-Chlor-2-nitrobenzoesäure wurden analog Beispiel 4 e) 45 g Rohprodukt erhalten.
b) 2-Acetyl-7-chlor-3-hydroxychinolin
   Aus 44 g (0,15 Mol) der obigen Verbindung wurden analog Beispiel 4 d) nach Chromatographie mit n-Heptan/Ethylacetat (2:1) an Kieselgel 18,3 g Produkt erhalten, Fp. 97-99°C (aus Petrolether). Weiterhin wurden 2,6 g des entsprechenden N-Oxids erhalten, Fp. 191°C (aus Diisopropylether).
c) 2-Acetyl-3-benzyloxy-7-chlorchinolin
   7,5 g (34 mMol) der vorstehenden Substanz wurden in 120 ml Diethylketon mit 4,7 g (34 mMol) Kaliumcarbonat und 4,1 ml (34 mMol) Benzylbromid umgesetzt. Man erhielt nach Chromatographie mit n-Heptan/Ethylacetat (4:1) an Kieselgel 8 g Produkt, Fp. 76-77°C (aus Petrolether).
d) 3-Benzyloxy-7-chlorchinolin-2-carbonsäure
   Aus 4,7 g (15 mMol) der vorstehenden Acetylverbindung wurden nach Haloform-Oxidation (NaOH/Wasser/Brom/Dioxan) 4,1 g Produkt erhalten, Fp. 140°C (u. Zers., aus Wasser).
e) N-((3-Benzyloxy-7-chlorchinolin-2-yl)carbonyl)glycinethylester
   Aus 3,2 g (10 mMol) der vorstehenden Carbonsäure, 1,4 g (10 mMol) Glycinethylester-Hydrochlorid, 3,8 ml (30 mMol) NEM, 1,5 g (11 mMol) HOBT und 4,3 g (10 mMol) CMC wurden analog zu Beispiel 4 g) 3,5 g Produkt erhalten, Fp. 138-140°C (aus Diisopropylether).
f) N-((3-Benzyloxy-7-chlorchinolin-2-yl)carbonyl)glycin
   2,0 g (5,0 mMol) des obigen Glycinethylesters wurden in 150 ml 1,5 N methanolischer NaOH verseift. Man erhielt 1,3 g Produkt, Fp. 105-108°C (sintern 70°C, schäumen, aus Diisopropylether).
g) Die Titelverbindung wurde erhalten, indem 1,2 g (3,2 mMol) der obigen Benzylverbindung in 60 ml 48 %igen wäßrigen Bromwasserstoff eingetragen und 45 min bei 80°C gerührt wurden. Nach dem Abkühlen engte man im Vakuum ein, wusch den Rückstand dreimal mit je 50 ml Tetrahydrofuran und erhält 0,57 g Produkt, Fp. 268-270°C.

### Beispiel 3

### N-((7-Chlor-3-hydroxy-6-(2,2,2-trifluorethyloxy)chinolin-2-yl)carbonyl)glycin

a) 4,5-Dichlor-2-nitrotoluol
   3,4-Dichlortoluol wurde mit konzentrierter Schwefelsäure und rauchender Salpetersäure (d=1,52) nitriert.
b) 4-Chlor-2-nitro-5-(2,2,2-trifluorethyloxy)toluol
   60 ml Trifluorethanol wurden unter Rühren und Kühlung portionsweise mit 40,4 g (360 mMol) Kalium-tert.butylat versetzt und auf 80-90°C erwärmt. Nach dem Abkühlen wurden 14,4 g (70 mMol) 4,5-Dichlor-2-nitrotoluol zugegeben und 1. h bei 105°C gerührt. Man engte im Vakuum ein, behandelte den Rückstand mit 300 ml Wasser und erhält nach dem Absaugen 12 g Rohprodukt, die mit n-Heptan/Ethylacetat an Kieselgel gereinigt wurden, 10 g Produkt, Fp. 76-79°C.
c) 4-Chlor-2-nitro-5-(2,2,2-trifluorethyloxy)benzoesäure
   10 g der vorstehenden Verbindung wurden in Pyridin/Wasser (1 :2) mit 85 g Kaliumpermanganat oxidiert; 8,2 g Produkt, Fp. 153-155°C (aus wäßriger Salzsäure).
d) 3-(4-Chlor-2-nitro-5-(2,2,2-trifluorethyloxy)benzoyl)acetylaceton
   Aus 10 g (34 mMol) der-vorstehenden Säure wurden analog Beispiel 4c) 10,3 g Produkt erhalten, Fp. 138-140°C (aus Petrolether).
e) 2-Acetyl-7-chlor-3-hydroxy-6-(2,2,2-trifluorethyloxy)chinolin
   10,2 g (26,6 mMol) der vorstehenden Verbindung wurden in 100 ml10%iger wäßriger KoH bei 50-70°C umgesetzt. Nach dem Ansäuern mit halbkonzentrierter HCL wurden 10 g Rohprodukt mit n-Heptan/Ethylacetat (4:1) an Kieselgel gereinigt; 4,2 g Produkt, Fp. 104-105°C.
f) 2-Acetyl-3-benzyloxy-7-chlor-6-(2,2,2-trifluorethyloxy)chinolin
   4,2 g (13 mMol) der vorstehenden Verbindung wurden in 100 ml Diethylketon mit 1,8 g (13 mMol) Kaliumcarbonat und 1,6 ml (13 mMol) Benzylbromid umgesetzt, 4,4 g Produkt, Fp. 110-112°C (aus Wasser).
g) 3-Benzyloxy-7-chlor-6-(2,2,2-trifluorethyloxy)chinolin-2-carbonsäure-Natriumsalz
   3,3 g (8 mMol) der vorstehenden Acetylverbindung wurden in wäßriger NaOH/Brom/1.4-Dioxan oxidiert. Nach dem Versetzen mit wäßriger Natriumpyrosulfit-(Na₂S₂O₅)-Lösung saugte man das ausgefallene Produkt ab und erhielt 3,6 g Natriumsalz, Fp. > 325°C.
h) N-((3-Benzyloxy-7-chlor-6-(2,2,2-trifluorethyloxylchinolin-2-yl)carbonyl)-glycinbenzylester
   3,3 g (8 mMol) der vorstehenden Carbonsäure wurden analog Beispiel 4 g) mit Glycinbenzylester-Tosylat umgesetzt; 2,92 g Produkt, das weiter umgesetzt wurde.
i) N-((3-Hydroxy-7-chlor-6-(2,2,2-trifluorethyloxy)chinolin-2-yl)carbonyl)-glycin-Tetrahydrofuran-Komplex (0.5 Äquivalente)
   1,12 g (2 mMol) der vorstehenden Benzylverbindung wurden in Tetrahydrofuran gelöst und in der Schüttelente mit Pd/C (10%) hydriert. Es wurde 0,58 g farbloses Produkt erhalten, Fp. 204-206°C (aus Petrolether), das laut ¹H-NMR 0.5 Äquivalente Tetrahydrofuran enthält.

### Beispiel 4

### N-((7-Chlor-3-hydroxy-6-(2,2,3,3,3-pentafluorpropyloxy)chinolin-2-yl)carbonyl)glycin

### Beispiel 5

### N-((7-Chlor-6-(2,2,3,3,4,4,4-heptafluorbutyloxy)-3-hydroxychinolin-2-yl)carbonyl)glycin

a) 4-Chlor-2-nitro-5-(2,2,3,3,4,4,4-heptafluorbutyloxy)toluol wurde analog Beispiel 31 b) mit Heptafluorbutanol erhalten, öliges Rohprodukt.
b) 4-Chlor-5-(2,2,3,3,4,4,4-heptafluorbutyloxy)-2-nitrobenzoesäure, analog Beispiel 31c), Fp. 115-117°C (aus Petrolether).
c) 3-(4-Chlor-5-(2,2,3,3,4,4,4-heptafluorbutyloxy)-2-nitrobenzoyl)-acetylaceton
   Analog Beispiel 31 d) und 4 c) wurden aus 7,7 g (19 mMol) der vorstehenden Säure 7,77g Produkt erhalten, Fp. 80-81 °C (aus Petrolether).
d) 2-Acetyl-7-chlor-6-(2,2,3,3,4,4,4-heptafluorbutyloxy)-3-hydroxychinolin wurde aus 7,7 g der vorstehenden Verbindung analog Beispiel 31 e) erhalten, nach Chromatographie mit n-Heptan/Ethylacetat (4:1) an Kieselgel 2,9 g öliges Produkt.
e) 2-Acetyl-3-benzyloxy-7-chlor-6-(2,2,3,3,4,4,4-heptafluorbutyloxy)chinolin
   Aus 2,9 g (6,9 mMol) der vorstehenden Verbindung wurden analog Beispiel 31 f) 3,8 g öliges Produkt erhalten.
f) 3-Benzyloxy-7-chlor-6-(2,2,3,3,4,4,4-heptafluorbutyloxy)chinolin-2-carbonsäure
   Aus 3,6 g (7 mMol) der vorstehenden Acetylverbindung wurden analog Beispiel 31 g) 3,4 g Produkt erhalten, Fp. 183-185°C (aus Petrolether).
g) N-((3-Benzyloxy-7-chlor-6-(2,2,3,3,4,4,4-heptafluorbutyloxy)chinolin-2-yl)carbonyl)glycinbenzylester
h) 3,3 g (6,5 mMol) der vorstehenden Carbonsäure wurden analog Beispiel 4 g) mit Glycinbenzylester-Tosylat umgesetzt. Nach Reinigung des Rohprodukts mit n-Heptan/Ethylacetat (2:1) an Kieslegel erhielt man 3,3 g öliges Produkt.
i) 1,32 g (2 mMol) der vorstehenden Verbindung wurden analog Beispiel **31** i) hydriert; 0,72 g farbloses Produkt, Fp. 172-173°C (aus Petrolether).

## Patentansprüche

1. Verbindungen der allgemeinen Formel (I) 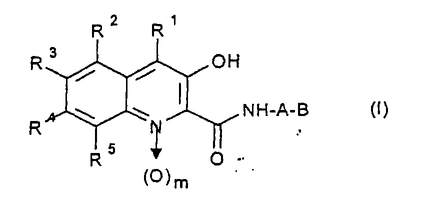 in welcher
m 0
A eine -CH₂-Gruppe, die mit einer Methylgruppe substituiert sein kann,
B -CO₂H,
R¹ Wasserstoff, (C₁-C₄)-Alkyl, (C₁-C₄)-Aloxy, Chlor, Brom,
R⁵ Wasserstoff, Fluor, Chlor, Methyl bedeutet,
R² und R³ gleich oder verschieden sind und Wasserstoff, (C₁-C₁₈)-Alkyl, (C₂-C₁₈)-Alkenyl, (C₂-C₁₈)-Alkinyl, Phenyl, Chlor, Brom, Hydroxy, Triflormethyl, (C₁-C₁₈)-Alkylsulfinyl, (C₁-C₁₈)-Alkylsulfonyl, Phenylsulfinyl, Phenylsulfonyl, Naphtylsulfinyl, Naphtylsulfonyl, (C₁-C₁₈)-Alkoxy, (C₃-C₈)-Cycloalkxy, (C₁-C₈)-Alkoxy- (C₁-C₈)-alkoxy, -O-[CH₂]ₓ-C_{f}H_{(2f+1-g)}F_{g}, Phenyl- (C₁-C₄)-alkoxy, Phenoxy, (C₁-C₁₂)-Alkanoyl, Phenyl- (C₁-C₄)-alkanoyl oder Benzoyl bedeuten, wobei in Substituenten mit einem Phenyl- oder Naphtyl- Ring dieser gegebenenfalls bis zu 5 gleiche oder verschiedene Substituenten aus der Reihe Fluor, Chlor, Brom, Nitril, Trifluormetyl, (C₁-C₆)-Alkyl, (C₁-C₆)-Alkoxy, -O-[CH₂]ₓ-C_{f}H_{(2f+1-g)}F_{g} oder (C₁-C₆)-Alkylsulfonyl trägt,
R⁴ Flour, Chlor, Brom bedeutet
f 1 - 8
g 0, 1 bis (2f+1)
x 0 bis 3 bedeuten, einschließlich der physiologisch wirksamen Salze.

2. Verbindungen der Formel I nach Anspruch 1, in der R¹ und R⁵ Wasserstoff bedeuten.

3. Verbindungen der Formel I nach Anspruch 2, in der einer der Substituenten R² oder R³ Wasserstoff bedeutet und der andere Wasserstoff, (C₁-C₁₆)-Alkyl, Fluor, Chlor, Brom, Trifluormethyl, (C₁-C₁₆)-Alkylsulfonyl, Phenylsulfonyl, (C₁-C₁₆)-Alkoxy, (C₁-C₈)-Alkoxy-(C₁-C₈)-alkoxy, -O-[CH₂]ₓ-C_{f}H_{(2f+1-g)}F_{g}, Benzyloxy oder Phenoxy bedeuten, wobei in Subtituenten, die einen Phenylring enthalten, dieser gegebenenfalls bis zu 3 Substituenten aus der Reihe Fluor, Chlor, Brom, Trifluormethyl, (C₁-C₆)-Alkyl, (C₁-C₆)-Alkoxy, -O-[CH₂]ₓ-C_{f}H_{(2f+1-g)}F_{g} oder (C₁-C₄)- Alkylsulfonyl trägt.

4. Verbindungen der Formel I nach Anspruch 3, in der R² Wasserstoff bedeutet.

5. Verbindungen der Formel I nach Anspruch 4, in der R³ Wasserstoff bedeutet.

6. Verbindungen der Formel I nach Anspruch 2, in der
R² (C₁-C₁₈)-Alkyl, (C₁-C₁₈)-Alkenyl, Phenyl, Chlor, Fluor, Brom, Trifluormethyl, (C₁-C₁₈)-Alkylsulfinyl, (C₁-C₁₈)-Alkylsulfonyl, Phenylsulfinyl, Phenylsulfonyl, Naphtylsulfinyl, Naphtylsulfonyl, (C₁-C₁₈)-Alkoxy, (C₃-C₈)-Cycloalkoxy, (C₁-C₈)-Alkoxy-(C₁-C₈)-alkoxy, -O-[CH₂]ₓ-C_{f}H_{(2f+1-g)}F_{g}, Phenyl-(C₁-C₄)-alkoxy, Phenoxy, (C₁-C₁₂)-Alkanoyl, Phenyl- (C₁-C₄)-alkanoyl oder Benzoyl bedeuten, wobei in Substituenten mit einem Phenyl- oder Naphtyl-Ring dieser gegebenenfalls bis zu 5 gleiche oder verschiedene Substituenten aus der Reihe Fluor, Chlor, Brom, Nitril, Trifluormethyl, (C₁-C₆)-Alkyl, (C₁-C₆)-Alkoxy, -O-[CH₂]ₓ-C_{f}H_{(2f+1-g)}F_{g} oder (C₁-C₆)-Alkylsulfonyl trägt, und
R³ (C₁-C₁₈)-Alkyl, (C₁-C₁₈)-Alkenyl, Phenyl, Flour, Trifluormethyl, (C₁-C₁₈)-Alkylsulfinyl, (C₁-C₁₈)-Alkylsulfonyl, Phenylsulfinyl, Naphtylsulfinyl, Naphtylsulfonyl, (C₁₁-C₁₈)-Alkoxy, (C₃-C₈)-Cycloalkoxy, (C₁-C₈)-Alkoxy- (C₁-C₈)-alkoxy, -O-[CH₂]ₓ-C_{f}H_{(2f+1-g)}F_{g}, außer Trifluoretoxy, Pentaflourpropoxy und Heptafluorbutoxy, Phenyl-(C₂-C₄)-alkoxy, Phenoxy, (C₁-C₁₂)-Alkanoyl, Phenyl- (C₁-C₄)-alkanoyl oder Benzoyl bedeuten, wobei in Substituenten mit einem Phenyl- oder Naphtyl-Ring dieser gegebenenfalls bis zu 5 gleiche oder verschiedene Substituenten aus der Reihe Fluor, Chlor, Brom, Nitril, Trifluormethyl, (C₁-C₆)-Alkyl, (C₁-C₆)-Alkoxy, -O-[CH₂]ₓ-C_{f}H_{(2f+1-g)}F_{g} oder (C₁-C₆) Alkylsulfonyl trägt.

7. Verbindungen der Formel I nach Anspruch 6, in der R² Wasserstoff bedeutet.

8. Verbindungen der Formel I nach Anspruch 7 in der R³ Wasserstoff bedeutet.

9. Verfahren zur Herstellung von Verbindungen der Formel I gemäß den Ansprüchen 1 - 8, **dadurch gekennzeichnet, dass**
1.i1.) Chinolin-2-carbonsäuren der Formel II (R¹¹=H) mit den Aminoestern der Formel III zu den Amidestern der Formel IV umgesetzt werden, oder
1.i2.) Chinolin-2-carbonsäureester der Formel II (R¹ =niedrig Alkyl) unter den Bedingungen der Aminolyse zu den Verbindungen der Formel IV umgesetzt werden; wobei R¹⁰ = PG (Protecting Group) und A-B= (CH₂)₁₋₄-CO₂H bedeutet 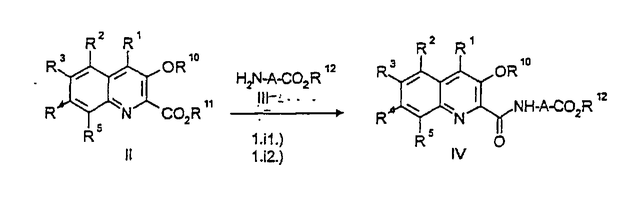
1.ii) die Verbindungen der Formeln I bzw. V aus ihren Estern der Formeln IV bzw. VI freigesetzt werden, und/oder 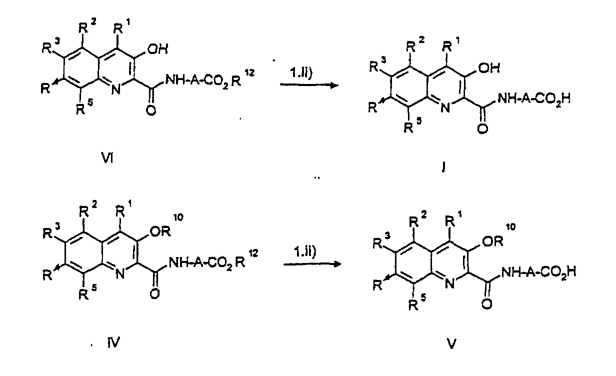
1.iii) die Verbindungen der Formeln I bzw. VI aus den Verbindungen der Formeln V bzw. IV durch Abspaltung der Hydroxy-Schutzgruppe R¹⁰ erhalten werden und ggf. 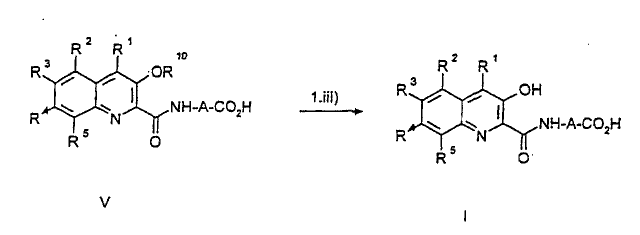
1.iv) die Verbindungen der Formel I, IV, V oder VI zu Verbindungen der Formeln Ia, IVa, Va oder VIa oxidiert werden.

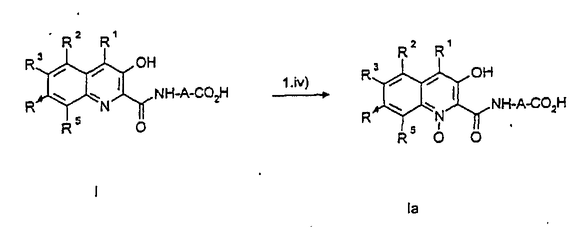

10. Verwendung von Verbindungen gemäß Ansprüchen 1 - 8 zur Herstellung eines Arzneimittels zur Hemmung der Kollagenbiosynthese.

11. Verwendung von Verbindungen gemäß den Ansprüchen 1 - 8 zur Herstellung eines Arzneimittels zur Inhibition der Prolylhydroxylase.

12. Verwendung von Verbindungen gemäß den Ansprüchen 1 - 8 zur Herstllung von Fibrosupressiva.

13. Verwendung der Verbindungen gemäß den Ansprüchen 1 - 8 zur Herstellung eines Arzneimittels gegen fibrotische Erkrankungen.

14. Verwendung der Verbindungen gemäß den Ansprüchen 1 - 8 zur Herstellung eines Arzneimittels gegen fibrotische Erkrankungen der Leber.

15. Verwendung von Verbindungen gemäß den Ansprüchen 1 - 8 zur Herstellung eines Arzneimittels gegen fibrotische Erkrankungen der Lunge.

16. Verwendung von Verbindungen gemäß den Ansprüchen 1 - 8 zur Herstellung eines Arzneimittels gegen fibrotische Erkrankungen der Haut.

17. Verwendung von Verbindungen der Formel I gemäß den Ansprüchen 1 - 8 zur Herstellung eines Arzneimittels zur Inhibition der Prolyl-hydroxylase und als Fibrosuppressiva.

18. Arzneimittel, enthaltend Verbindungen der Formel I gemäß den Ansprüchen 1 - 8.

19. Zwischenprodukte der Formel II 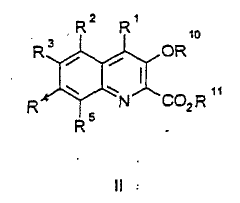 in der
R¹ - R⁵ die Bedeutungen haben, die für die Verbindungen der Formel I gemäß den Ansprüchen 1 - 8 gelten,
R¹⁰ Wasserstoff oder eine HO-Schutzgruppe ist und
R¹¹ Wasserstoff, (C₁-C₈)-Alkyl und Benzyl bedeuten.

20. Zwischenprodukte der Formel X 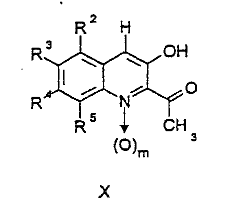 in der
m 0 oder 1 und
R² - R⁵ die Bedeutungen haben, die für die Verbindungen der Formel I gemäß den Ansprüchen 1 - 8 gelten.

## Claims

1. Compounds of the formula (I) 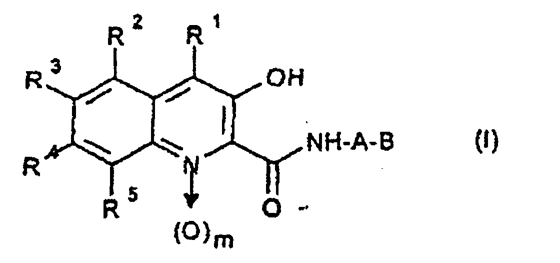 in which
m is 0,
A is a -CH₂- group, which can be substituted by a methyl group,
B is -CO₂H,
R¹ is hydrogen, (C₁-C₄)-alkyl, (C₁-C₄)-alkoxy, chlorine or bromine,
R⁵ is hydrogen, fluorine, chlorine or methyl and
R² and R³ are identical or different and are hydrogen, (C₁-C₁₈)-alkyl, (C₂-C₁₈)-alkenyl, (C₂-C₁₈)-alkynyl, phenyl, chlorine, bromine, hydroxyl, trifluoromethyl, (C₁-C₁₈)-alkylsulphinyl, (C₁-C₁₈)-alkylsulphonyl, phenylsulphinyl, phenylsulphonyl, naphthylsulphinyl, naphthylsulphonyl, (C₁-C₁₈)-alkoxy, (C₃-C₈)-cycloalkoxy, (C₁-C₈)-alkoxy-(C₁-C₈)-alkoxy, -O-[CH₂]ₓ-C_{f}H_{(2f+1-g)}F_{g}, phenyl- (C₁-C₄)-alkoxy, phenoxy, (C₁-C₁₂)-alkanoyl, phenyl-(C₁-C₄)-alkanoyl or benzoyl, where, in substituents with a phenyl or naphthyl ring, this optionally carries up to 5 identical or different substituents from the series consisting of fluorine, chlorine, bromine, nitrile, trifluoromethyl, (C₁-C₆)-alkyl, (C₁-C₆)-alkoxy, - O-[CH₂]ₓ-C_{f}H_{(2f+1-g)}F_{g} or (C₁-C₆)-alkylsulphonyl,
R⁴ is fluorine, chlorine or bromine,
f is 1 to 8,
g is 0, 1 to (2f + 1),
x is 0 to 3 and physiologically active salts thereof.

2. Compounds of the formula I according to Claim 1, in which R¹ and R⁵ are hydrogen.

3. Compounds of the formula I according to Claim 2, in which one of the substituents R² or R³ is hydrogen and the other is hydrogen, (C₁-C₁₆)-alkyl, fluorine, chlorine, bromine, trifluoromethyl, (C₁-C₁₆)-alkylsulphonyl, phenylsulphonyl, (C₁-C₁₆)-alkoxy, (C₁-C₈)-alkoxy-(C₁-C₈)-alkoxy, -O-[CH₂]ₓ-C_{f}H_{(2f+1-g)}F_{g}, benzyloxy or phenoxy, where, in substituents which contain a phenyl ring, this optionally carries up to 3 substituents from the series consisting of fluorine, chlorine, bromine, trifluoromethyl, (C₁-C₆)-alkyl, (C₁-C₆)-alkoxy, -O-[CH₂]ₓ-C_{f}H_{(2f+1-g)}F_{g} or (C₁-C₄)-alkylsulphonyl.

4. Compounds of the formula I according to Claim 3, in which R² is hydrogen.

5. Compounds of the formula I according to Claim 4, in which R³ is hydrogen.

6. Compounds of the formula I according to Claim 2, in which
R² is (C₁-C₁₈)-alkyl, (C₁-C₁₈)-alkenyl, phenyl, chlorine, fluorine, bromine, trifluoromethyl, (C₁-C₁₈)-alkylsulphinyl, (C₁-C₁₈)-alkylsulphonyl, phenylsulphinyl, phenylsulphonyl, naphthylsulphinyl, naphthylsulphonyl, (C₁-C₁₈)-alkoxy, (C₃-C₈)-cycloalkoxy, (C₁-C₈)-alkoxy-(C₁-C₈)-alkoxy, -O-[CH₂]ₓ-C_{f}H_{(2f+1-g)}F_{g}, phenyl-(C₁-C₄)-alkoxy, phenoxy, (C₁-C₁₂)-alkanoyl, phenyl-(C₁-C₄)-alkanoyl or benzoyl, where, in substituents having a phenyl or naphthyl ring, this optionally carries up to 5 identical or different substituents from the series consisting of fluorine, chlorine, bromine, nitrile, trifluoromethyl, (C₁-C₆)-alkyl, (C₁-C₆)-alkoxy, - O-[CH₂]ₓ-C_{f}H_{(2f+1-g)}F_{g} and (C₁-C₆)-alkylsulphonyl, and
R³ is (C₁-C₁₈)-alkyl, (C₁-C₁₈)-alkenyl, phenyl, fluorine, trifluoromethyl, (C₁-C₁₈)-alkylsulphinyl, (C₁-C₁₈)-alkylsulphonyl, phenylsulphinyl, naphthylsulphinyl, naphthylsulphonyl, (C₁₁-C₁₈)-alkoxy, (C₃-C₈)-cycloalkoxy, (C₁-C₈)-alkoxy-(C₁-C₈)-alkoxy, -O-[CH₂]ₓ-C_{f}H_{(2f+1-g)}F_{g}, apart from trifluoroethoxy, pentafluoropropoxy and heptafluorobutoxy, phenyl-(C₂-C₄)-alkoxy, phenoxy, (C₁-C₁₂)-alkanoyl, phenyl-(C₁-C₄)-alkanoyl or benzoyl, where, in substituents having a phenyl or naphthyl ring, this optionally carries up to 5 identical or different substituents from the series consisting of fluorine, chlorine, bromine, nitrile, trifluoromethyl, (C₁-C₆)-alkyl, (C₁-C₆)-alkoxy, -O-[CH₂]ₓ-C_{f}H_{(2f+1-g)}F_{g} and (C₁-C₆)-alkylsulphonyl.

7. Compounds of the formula I according to Claim 6, in which R² is hydrogen.

8. Compounds of the formula I according to Claim 7, in which R³ is hydrogen.

9. Process for the preparation of compounds of the formula I according to Claim 8, **characterized in that** it comprises
1.i1.) reacting quinoline-2-carboxylic acids of the formula II (R¹¹ = H) with the amino esters of the formula III to give the amide esters of the formula IV, or
1.i2.) reacting quinoline-2-carboxylic acid esters of the formula II (R¹ = lower alkyl) under the conditions of aminolysis to give the compounds of the formula IV; where R¹⁰ is PG (Protecting Group) and A-B is (CH₂)₁₋₄-CO₂H 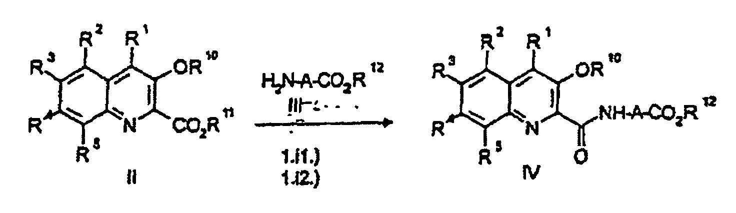
1.ii) liberating the compounds of the formula I or V from their esters of the formula IV or VI; and/or 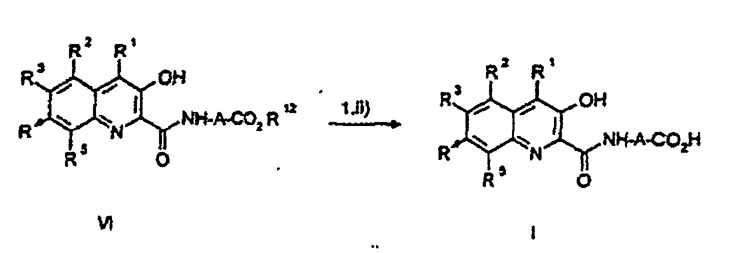 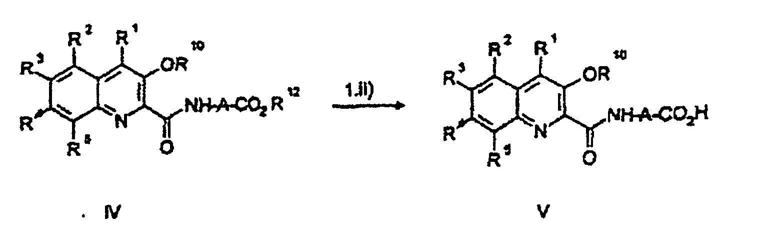
1.iii) obtaining the compounds of the formula I or VI from the compounds of the formula V or IV by splitting off the hydroxyl-protecting group R¹⁰, and if appropriate 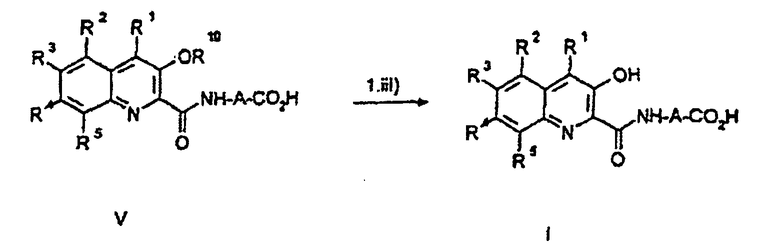
1.iv) oxidizing the compounds of the formula I, IV, V or VI to give compounds of the formula Ia, IVa, Va or VIa.

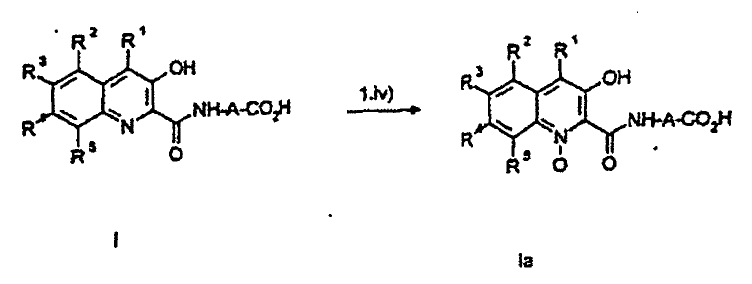

10. Use of compounds according to Claims 1 to 8 for the preparation of a medicament for inhibiting collagen biosynthesis.

11. Use of compounds according to Claims 1 to 8 for the preparation of a medicament for inhibiting prolyl hydroxylase.

12. Use of compounds according to Claims 1 to 8 for the preparation of fibrosupressants.

13. Use of compounds according to Claims 1 to 8 for the preparation of a medicament against fibrotic diseases.

14. Use of compounds according to Claims 1 to 8 for the preparation of a medicament against fibrotic diseases of the liver.

15. Use of compounds according to Claims 1 to 8 for the preparation of a medicament against fibrotic diseases of the lung.

16. Use of compounds according to Claims 1 to 8 for the preparation of a medicament against fibrotic diseases of the skin.

17. Use of compounds of the formula I according to Claims 1 to 8 for the preparation of a medicament for inhibiting prolyl hydroxylase and as fibrosupressants.

18. Medicament comprising compounds of the formula I according to Claims 1 to 8.

19. Intermediate products of the formula II 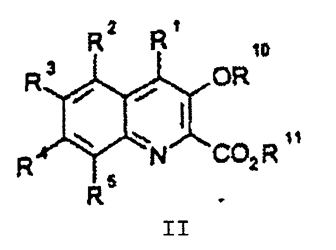 in which
R¹ to R⁵ have the meanings which apply to the compounds of the formula I according to Claims 1 to 8,
R¹⁰ is hydrogen or an HO-protecting group and
R¹¹ is hydrogen, (C₁-C₈)-alkyl or benzyl.

20. Intermediate products of the formula X 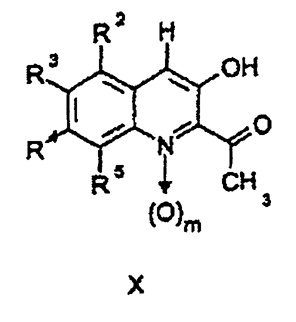 in which
m is 0 or 1, and
R² to R⁵ have the meanings which apply to the compounds of the formula I according to Claims 1 to 8.

## Revendications

1. Composés de la formule générale (I) : 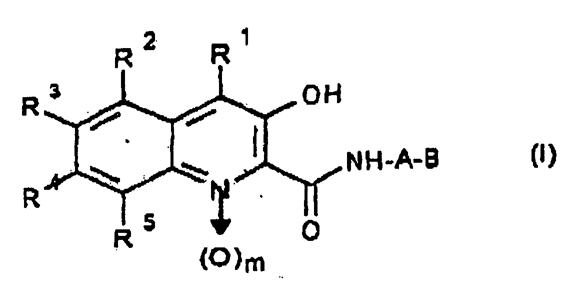 dans laquelle :
m représente 0 ;
A représente un radical -CH₂-, qui peut être substitué avec un radical méthyle ;
B représente -COOH ;
R¹ représente hydrogène, alkyle (C₁-C₄), alcoxy (C₁-C₄), chlore, brome ;
R⁵ représente hydrogène, fluor, chlore, méthyle ;
R² et R³ sont identiques ou différents et représentent hydrogène, alkyle (C₁-C₁₈), alcényle (C₂-C₁₈), alcynyle (C₂-C₁₈), phényle, chlore, brome, hydroxyle, trifluorométhyle, alkylsulfinyle (C₁-C₁₈), alkylsulfonyle (C₁-C₁₈), phénylsulfinyle, phénylsulfonyle, naphtylsulfinyle, naphtylsulfonyle, alcoxy (C₁-C₁₈), cycloalkoxy (C₃-C₈), alcoxy (C₁-C₈)-alcoxy (C₁-C₈), -O-(CH₂)ₓ-C_{f}H_{(2f+1-g)}F_{g}, phénylalcoxy (C₁-C₄),phénoxy, alcanoyle (C₁-C₁₂), phénylalcanoyle (C₁-C₄) ou benzoyle, où pour les substituants avec un cycle phényle ou naphtyle, ceux-ci portent le cas échéant, jusqu'à 5 substituants identiques ou différents du groupe fluor, chlore, brome, nitrile, trifluorométhyle, alkyle (C₁-C₆), alcoxy (C₁-C₆), -O-(CH₂)ₓ-C_{f}H_{(2f+1-g)}F_{g} ou alkylsulfonyle (C₁-C₆) ;
R⁴ représente fluor, chlore, brome ;
f représente 1-8 ;
g représente 0, 1 à (2f+1) ;
x représente 0 à 3, y inclus les sels physiologiquement actifs.

2. Composés de la formule I selon la revendication 1, dans laquelle R¹ et R⁵ signifient hydrogène.

3. Composés de la formule I selon la revendication 2, dans laquelle un des substituants R² ou R³ représente hydrogène et l'autre, hydrogène, alkyle (C₁-C₁₆), fluor, chlore, brome, trifluorométhyle, alkylsulfonyle (C₁-C₁₆), phénylsulfonyle, alcoxy (C₁-C₁₆), alcoxy (C₁-C₈)-alcoxy (C₁-C₈), -O-(CH₂)ₓ-C_{f}H_{(2f+1-g)}F_{g}, benzyloxy ou phénoxy, où pour les substituants avec un cycle phényle, celui-ci porte le cas échéant, jusqu'à 3 substituants du groupe fluor, chlore, brome, trifluorométhyle, alkyle (C₁-C₆), alcoxy (C₁-C₆), -O-(CH₂)ₓ-C_{f}H_{(2f+1-g)}F_{g} ou alkylsulfonyle (C₁-C₄).

4. Composés de la formule I selon la revendication 3, dans laquelle R² signifie hydrogène.

5. Composés de la formule I selon la revendication 4, dans laquelle R³ signifie hydrogène.

6. Composés de la formule I selon la revendication 2, dans laquelle
R² représente alkyle (C₁-C₁₈), alcényle (C₂-C₁₈), phényle, chlore, fluor, brome, trifluorométhyle, alkylsulfinyle (C₁-C₁₈), alkylsulfonyle (C₁-C₁₈), phénylsulfinyle, phénylsulfonyle, naphtylsulfinyle, naphtylsulfonyle, alcoxy (C₁-C₁₈), cycloalcoxy (C₃-C₈), alcoxy (C₁-C₈)-alcoxy (C₁-C₈), -O-(CH₂)ₓ-C_{f}H_{(2f+1-g)}F_{g}, phénylalcoxy (C₁-C₄), phénoxy, alcanoyle (C₁-C₁₂), phénylalcanoyle (C₁-C₄) ou benzoyle, où pour les substituants avec un cycle phényle ou naphtyle, ceux-ci portent le cas échéant, jusqu'à 5 substituants identiques ou différents du groupe fluor, chlore, brome, nitrile, trifluorométhyle, alkyle (C₁-C₆), alcoxy (C₁-C₆), -O-(CH₂)ₓ-C_{f}H_{(2f+1-g)}F_{g} ou alkylsulfonyle (C₁-C₆), et
R³ représente alkyle (C₁-C₁₈), alcényle (C₂-C₁₈), phényle, fluor, trifluorométhyle, alkylsulfinyle (C₁-C₁₈), alkylsulfonyle (C₁-C₁₈), phénylsulfinyle, naphtylsulfinyle, naphtylsulfonyle, alcoxy (C₁-C₁₈), cycloalcoxy (C₃-C₈), alcoxy (C₁-C₈)-alcoxy (C₁-C₈), -O-(CH₂)ₓ-C_{f}H_{(2f+1-g)}F_{g}, en dehors de trifluoroéthoxy, pentafluoropropoxy et heptafluorobutoxy, phénylalcoxy (C2-C₄), phénoxy, alcanoyle (C₁-C₁₂), phénylalcanoyle (C₁-C₄) ou benzoyle, où pour les substituants avec un cycle phényle ou naphtyle, ceux-ci portent le cas échéant, jusqu'à 5 substituants identiques ou différents du groupe fluor, chlore, brome, nitrile, trifluorométhyle, alkyle (C₁-C₆), alcoxy (C₁-C₆), -O-(CH₂)ₓ-C_{f}H_{(2f+1-g)}F_{g} ou alkylsulfonyle (C₁-C₆),

7. Composés de la formule I selon la revendication 6, dans laquelle R² signifie hydrogène.

8. Composés de la formule I selon la revendication 7, dans laquelle R³ signifie hydrogène.

9. Procédé de préparation des composés de la formule I selon les revendications 1-8, **caractérisé en ce que** :
1.i1) On fait réagir les acide quinoléine-2-carboxyliques de la formule II (R¹¹ = H) avec les aminoesters de la formule III en les amidoesters de la formule IV, ou
1.i2) On fait réagir les esters d'acide quinoléine-2-carboxylique de la formule II (R¹ = alkyle inférieur) dans des conditions d'aminolyse, en les composés de la formule IV, où R¹⁰ = PG (protecting group) et A-B = (CH₂)₁₋₄COOH : 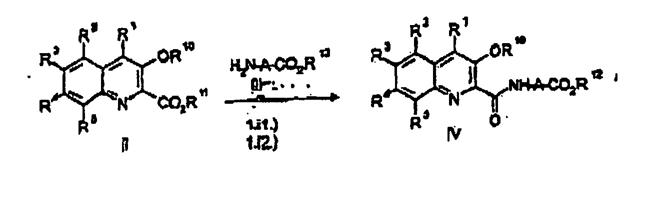
1.ii) les composés des formules I ou V sont libérés de leurs esters des formules IV ou VI : 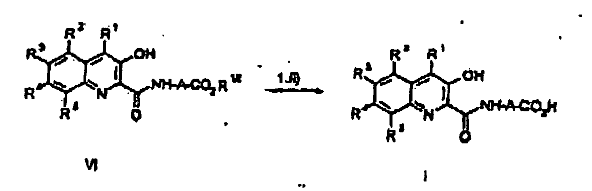 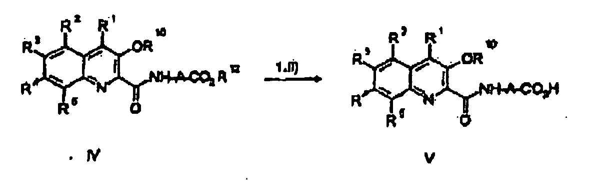 et/ou
1.iii) les composés des formules I ou VI sont obtenus à partir des composés V ou IV par clivage des groupes protecteurs d'hydroxyle R¹⁰ : 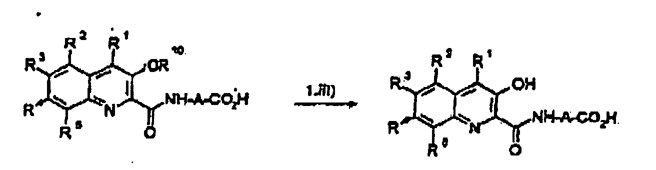 et le cas échéant,
1.iv) les composés des formules I, IV, V ou VI sont oxydés en les composés des formules Ia, IVa, Va ou VIa :

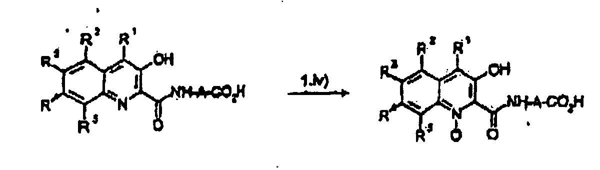

10. Utilisation des composés selon les revendications 1-8, pour la préparation d'un agent pharmaceutique pour inhiber la biosynthèse de collagène.

11. Utilisation des composés selon les revendications 1-8, pour la préparation d'un agent pharmaceutique pour inhiber la prolylhydroxylase.

12. Utilisation des composés selon les revendications 1-8, pour la préparation d'un fibrosuppresseur.

13. Utilisation des composés selon les revendications 1-8, pour la préparation d'un agent pharmaceutique contre les maladies fibrotiques.

14. Utilisation des composés selon les revendications 1-8, pour la préparation d'un agent pharmaceutique contre les maladies fibrotiques du foie.

15. Utilisation des composés selon les revendications 1-8, pour la préparation d'un agent pharmaceutique contre les maladies fibrotiques des poumons.

16. Utilisation des composés selon les revendications 1-8, pour la préparation d'un agent pharmaceutique contre les maladies fibrotiques de la peau.

17. Utilisation des composés de la formule I selon les revendications 1-8, pour la préparation d'un agent pharmaceutique pour inhiber la prolylhydroxylase et comme fibrosuppresseur.

18. Agent pharmaceutique, contenant des composés de la formule I selon les revendications 1-8.

19. Produits intermédiaire de la formule II : 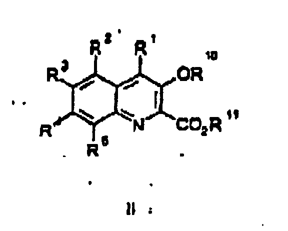 dans laquelle :
R¹-R⁵ ont les significations, qui valent pour les composés de la formule I selon les revendications 1-8,
R¹⁰ est hydrogène ou un groupe protecteur de OH, et
R¹¹ représente hydrogène, alkyle (C₁-C₈) et benzyle.

20. Produits intermédiaire de la formule X : 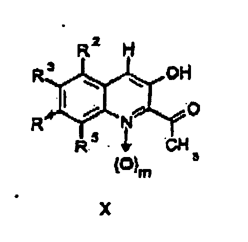 dans laquelle :
m est 0 ou 1, et
R¹-R⁵ ont les significations, qui valent pour les composés de la formule I selon les revendications 1-8.
